(19) Europäisches Patentamt
European Patent Office
Office européen des brevets

(11) EP 3 838 927 B1

(12) **FASCICULE DE BREVET EUROPEEN**

(45) Date de publication et mention de la délivrance du brevet:
**09.10.2024 Bulletin 2024/41**

(21) Numéro de dépôt: **20214618.9**

(22) Date de dépôt: **16.12.2020**

(51) Classification Internationale des Brevets (IPC):
***C08B 37/08*** *(2006.01)* ***C08L 5/08*** *(2006.01)*
***A61K 47/36*** *(2006.01)*

(52) Classification Coopérative des Brevets (CPC):
(C-Sets disponibles)
**C08B 37/0072; A61L 27/34; A61L 27/50; A61L 27/52; A61L 27/54; A61M 5/19; A61M 5/31596; C08L 5/08;** A61K 9/0024; A61K 9/06; A61K 47/36; A61L 2300/41; A61M 5/286

(Cont.)

(54) **ACIDE HYALURONIQUE RÉTICULÉ ET SON UTILISATION POUR LIMITER LA RÉCIDIVE D'UNE TUMEUR**

VERNETZTE HYALURONSÄURE UND IHRE VERWENDUNG ZUR BEGRENZUNG DES WIEDERAUFTRETENS EINES TUMORS

CROSS-LINKED HYALURONIC ACID AND USE THEREOF FOR LIMITING THE RECURRENCE OF A TUMOUR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.12.2019 FR 1914665**

(43) Date de publication de la demande:
**23.06.2021 Bulletin 2021/25**

(73) Titulaire: **COMMISSARIAT À L'ÉNERGIE ATOMIQUE ET AUX ÉNERGIES ALTERNATIVES**
**75015 Paris (FR)**

(72) Inventeurs:
- **TEXIER-NOGUES, Isabelle**
  **38054 GRENOBLE CEDEX 09 (FR)**
- **GAUDE, Christophe**
  **38054 GRENOBLE CEDEX 09 (FR)**
- **HOANG, Antoine**
  **38054 GRENOBLE CEDEX 09 (FR)**
- **LE NAOUR, Audrey**
  **38000 GRENOBLE (FR)**
- **RATEL, David**
  **38054 GRENOBLE CEDEX 09 (FR)**
- **VIGNOUD, Séverine**
  **38054 GRENOBLE CEDEX 09 (FR)**

(74) Mandataire: **Lavoix**
**2, place d'Estienne d'Orves**
**75441 Paris Cedex 09 (FR)**

(56) Documents cités:
- **JOHN G. HARDY ET AL: "Biodegradable hydrogels composed of oxime crosslinked poly(ethylene glycol), hyaluronic acid and collagen: a tunable platform for soft tissue engineering", JOURNAL OF BIOMATERIALS SCIENCE. POLYMER EDITION., vol. 26, no. 3, 2 January 2015 (2015-01-02), NL, pages 143 - 161, XP055722357, ISSN: 0920-5063, DOI: 10.1080/09205063.2014.975393**
- **ALEXANDER E. G. BAKER ET AL: "Independently Tuning the Biochemical and Mechanical Properties of 3D Hyaluronan-Based Hydrogels with Oxime and Diels-Alder Chemistry to Culture Breast Cancer Spheroids", BIOMACROMOLECULES, vol. 18, no. 12, 31 October 2017 (2017-10-31), pages 4373 - 4384, XP055722322, ISSN: 1525-7797, DOI: 10.1021/acs.biomac.7b01422**
- **GREGORY N. GROVER ET AL: "Oxime Cross-Linked Injectable Hydrogels for Catheter Delivery", ADVANCED MATERIALS, vol. 25, no. 21, 12 March 2013 (2013-03-12), pages 2937 - 2942, XP055684512, ISSN: 0935-9648, DOI: 10.1002/adma.201205234**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **TAKURO HOZUMI ET AL: "Injectable Hydrogel with Slow Degradability Composed of Gelatin and Hyaluronic Acid Cross-Linked by Schiff's Base Formation", BIOMACROMOLECULES, vol. 19, no. 2, 18 January 2018 (2018-01-18), pages 288 - 297, XP055639308, ISSN: 1525-7797, DOI: 10.1021/acs.biomac.7b01133**

(52) Classification Coopérative des Brevets (CPC): (Cont.)

C-Sets
**A61L 27/34, C08L 5/08**

## Description

La présente invention concerne un acide hyaluronique réticulé utile pour limiter la récidive d'une tumeur, de préférence du glioblastome, chez un patient ayant subi une intervention chirurgicale d'exérèse de la tumeur.

Les glioblastomes représentent environ 50 % de toutes les tumeurs intracrâniennes d'adulte. Plus de 50 % de ces tumeurs sont des formes malignes (astrocytomes anaplasiques et glioblastomes). Le glioblastome (astrocytome de grade IV) constitue la tumeur gliale la plus maligne. Chez l'adulte, les glioblastomes sont les tumeurs cérébrales les plus fréquentes avec une incidence de l'ordre de 4 nouveaux cas par an et pour 100.000 habitants. C'est la deuxième cause de mortalité des cancers chez l'enfant après la leucémie.

La chirurgie constitue le traitement de première intention des gliomes de haut grade (glioblastomes, astrocytomes anaplasiques...) et parfois de certains gliomes de bas grade.

Après résection tumorale, la récidive survient dans près de 90% des patients au niveau des berges d'exérèse. Cette récurrence locale est due au caractère infiltrant des gliomes. Quelque-soit l'étendue de l'exérèse chirurgicale, des cellules résiduelles de gliome restent dans les berges d'exérèse. Dans l'état actuel, la récidive post-exérèse dans la berge d'exérèse est quasi inévitable et invariablement fatale.

Le lien existant entre les phénomènes lésionnels et la promotion tumorale sont aujourd'hui bien établis. Cette association est liée entre autre au fait que l'inflammation chronique et l'angiogenèse post-lésionnelles agissent vraisemblablement comme des promoteurs tumoraux. Une des conséquences de la lésion chirurgicale serait paradoxalement de concourir à la récidive tumorale.

Les thérapies usuelles suivant l'intervention chirurgicale d'exérèse du glioblastome pour limiter/prévenir la récidive sont la radiothérapie accompagnée ou non d'une chimiothérapie systémique lourde. Différentes solutions ont été explorées pour remplacer la chimiothérapie systémique par une délivrance locale d'anticancéreux dans le site tumoral. Ces systèmes de délivrance localisée d'anticancéreux sont notamment :

- des implants polymériques tels que le pCPP :SA (co-polymère de 1,3-bis-(p-carboxyphenoxy)propane et acide sébacique (décanedioïque)) (Gliadel®), l'acide poly(lactique) (PLA), des co-polymères caprolactone-acide glycolique (PCL/PGA) ou acide poly(lactique-co-glycolique) (PLGA), des matériaux de type cellulosique, ces implants étant imprégnés d'anticancéreux. Ces matériaux restent rigides mécaniquement, ce qui empêche qu'ils comblent tout l'espace de la cavité tumorale.
- des hydrogels, qui sont des réseaux 3D polymériques capables d'absorber et d'emprisonner de très larges quantités d'eau. Ainsi, des hydrogels à base de PLGA (acide poly(lactique-co-glycolique)) (Oncogel®), des copolymères thermosensibles de type PNIPPAM-PEG (Mebiol® gel), poly(organophosphazene) ou chitosan-glycérophosphate, et de l'alginate réticulé ioniquement par $Ca^{2+}$ ont été décrits. Des hydrogels de chitosan-PEG (polyéthylène glycol) pour la délivrance locale de lymphocytes T encapsulés ont également été rapportés. Ces hydrogels sont également chargés en anticancéreux. Les hydrogels présentent l'avantage par rapport aux implants polymériques de remplir la cavité d'un matériau se rapprochant structurellement des tissus par leur forte contenance en tampon aqueux/fluide biologique. Le rôle de ces hydrogels est essentiellement d'augmenter la viscosité de la formulation d'anticancéreux injectée dans la cavité d'exérèse pour ralentir la diffusion de l'anticancéreux et ainsi prolonger la cinétique de délivrance. L'inconvénient des hydrogels décrits ci-dessus est leur faible cohésion en milieu plus dilué ou au cours du temps, puisque leur organisation en gel ne dépend que d'interactions faibles : les hydrogels listés ci-dessus sont des gels physiques, dans lesquels le réseau polymérique est maintenu cohésif par des enchevêtrements moléculaires, des interactions ioniques, des liaisons hydrogènes et/ou des interactions hydrophobes, et non des gels chimiques dans lesquels les chaînes polymériques sont liées les unes aux autres par liaison covalente.
- des gels chimiques, tel que des gels à base de PEG-méthacrylate réticulés in-situ (c'est à dire dans la cavité tumorale) par photopolymérisation, et des gels à base de PEG-diacrylate réticulés in-situ par réticulation chimique en présence de persulfate d'ammonium et de N,N,N,N-tetramethylethylenediamine. Les radicaux générés et les produits utilisés pour ce type de réaction présentent des risques potentiels de toxicité.

Quel que soit le matériau considéré, celui-ci est utilisé pour faire de la chimiothérapie locale. Ces stratégies thérapeutiques sont focalisées sur la destruction des cellules tumorales restantes grâce à la délivrance d'agents anticancéreux ou de lymphocytes T. On cherche donc à éviter la colonisation du gel par les cellules tumorales en particulier, et plus généralement par les cellules. Il y a donc un préjugé pour l'homme du métier à utiliser des polymères connus comme favorisant la migration cellulaire et la recolonisation par des cellules, tel que l'acide hyaluronique (HA).

A la connaissance des inventeurs, le seul article décrivant l'utilisation d'un gel à base d'acide hyaluronique réticulé en utilisant la chimie aldéhyde oxyamine pour des applications anticancéreuses est celui de Baker et al. (Biomacromolecules, 2017, 18, 4373-4384), utilisé pour la culture cellulaire de cellules tumorales de sein. Un dérivé d'acide hyaluronique a été obtenu par fonctionnalisation d'acide hyaluronique par du méthyl furane et de l'amino acetaldehyde diméthyl acétal, puis déprotection de la fonction aldéhyde, puis réticulation avec un agent de réticulation $H_2N$-O-

$CH_2$-PEG-$CH_2$-O-$NH_2$. Le squelette de l'acide hyaluronique est conservé, car les cycles des sucres des unités disaccharidiques de l'acide hyaluronique ne sont pas modifiés. Un premier inconvénient est que la préparation du dérivé d'acide hyaluronique porteur de fonctions aldéhydes nécessite deux étapes et des purifications. Un deuxième est que le procédé de préparation conduit à une dégradation de l'acide hyaluronique, puisque le procédé conduit à une diminution de 15% de son poids moléculaire. Un troisième est que l'agent de réticulation est à base de PEG, ceux-ci étant de plus en plus écartés du fait qu'ils sont susceptibles de conduire au développement d'anticorps anti-PEG et de provoquer des réactions allergiques.

Par ailleurs, des dérivés d'acide hyaluronique ont déjà été utilisés en médecine régénérative, à savoir pour la réparation du cerveau après un accident vasculaire cérébral (AVC) : des gels physiques comme des gels de HA/méthylcellulose, et des gels chimiques formés par réaction du HA avec le dihydrazide d'acide adipique en présence de 1-éthyl-3-(3-diméthylaminopropyl)carbodiimide (EDC) (qui a comme inconvénient majeur de présenter des risques d'interférence avec les fonctions amines des protéines des tissus), formés soit par réticulation chimique (réaction de Mickaël, chimie thiol-ène) entre un groupement méthacrylate ou acrylamide et un groupement thiol (qui présente également des risques d'interférence avec les protéines) ou soit par réticulation photochimique à partir de HA-méthacrylate en présence de photoinitiateur (risques de toxicité). Les dérivés d'acide hyaluronique utilisés après un AVC sont utilisés sous forme liquide. Là encore, ils servent de vecteur aux principes actifs.

Il existe un besoin thérapeutique de développer des méthodes pour prévenir la récidive d'une tumeur, de préférence du glioblastome, chez un patient ayant subi une intervention chirurgicale d'exérèse de la tumeur.

A cette fin, selon un premier objet, l'invention concerne une composition polymérique comprenant (ou consistant en) un polymère dérivé d'acide hyaluronique comprenant des unités réticulées pour former ensemble au moins l'un des groupements de formule (I), (II) ou (III) suivantes :

[Chem 1]

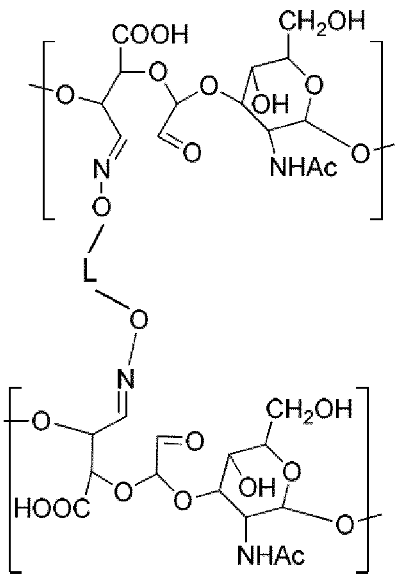

(I),

[Chem 2]

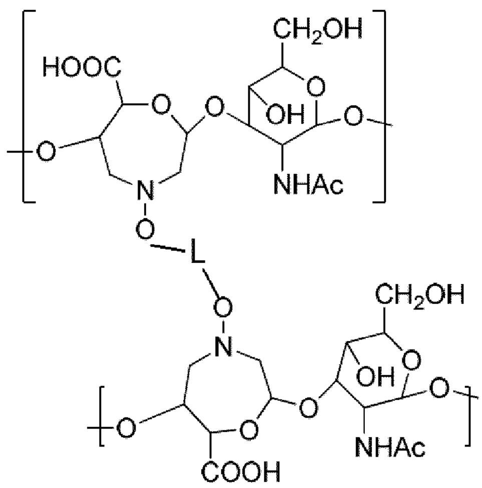

(II),

[Chem 3]

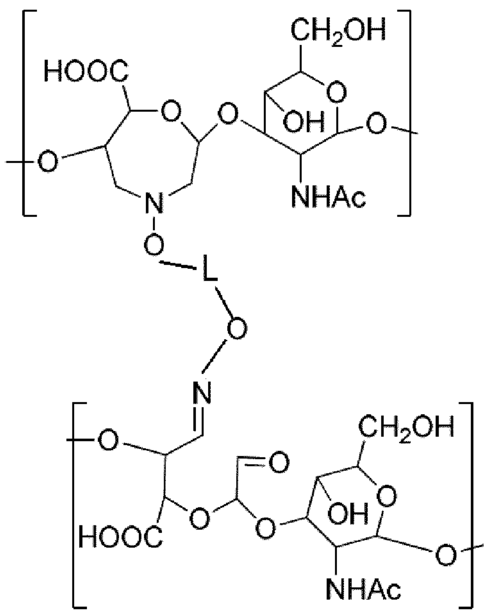

(III)

chaque groupement étant sous forme neutre ou de sel,
et dans lesquelles L est une chaîne hydrocarbonée comprenant de 2 à 10 atomes de carbone et éventuellement interrompue par un ou plusieurs groupes -O- ou -NH-.

Les groupements de formules (I), (II) et (III) sont structurellement très proches. Le groupement de formule (I) comprend deux formes ouvertes, celui de formule (II) deux formes cycliques, et celui de formule (III) une forme ouverte et une forme cyclique. Le polymère peut contenir un mélange de ces groupements. Lorsqu'il n'en comprend qu'un seul, il s'agit généralement du groupement de formule (II) car la forme cyclique est thermodynamiquement plus stable et donc favorisée.

L est un groupe divalent et représente une chaîne hydrocarbonée éventuellement interrompue par un ou plusieurs groupes -O- ou -NH-, et comprenant de 2 à 10 atomes de carbone, notamment de 3 à 8 atomes de carbone, de préférence de 4 à 6 atomes de carbone, en particulier 4 atomes de carbone. De préférence, quand ils sont présents, les groupes -O- et/ou -NH- ne sont pas adjacents les uns des autres. De préférence, quand ils sont présents, L comprend

au plus 8 groupes - O- et/ou -NH-, notamment au plus 4 groupes -O- et/ou -NH-, de préférence 1 groupe -O- ou -NH- ou 2 groupes choisis parmi -NH- et -O-.

De préférence, L représente un groupe de formule $-(CH_2)_2-O-(CH_2)_2-$, $-(CH_2)_2-O-(CH_2)_2-O-(CH_2)_2-$, $-(CH_2)_2-NH-(CH_2)_2-$, $-(CH_2)_2-NH-(CH_2)_2-NH-(CH_2)_2-$, $-(CH_2)_2-NH-(CH_2)_2-O-(CH_2)_2-$ ou $-(CH_2)_p-$ dans lequel p est un nombre entier de 2 à 10, le groupe $-(CH_2)_2-O-(CH_2)_2-$ étant particulièrement préféré.

Lorsque les groupements, groupes, unités sont sous forme de sel, ils sont de préférence des sels pharmaceutiquement acceptables.

Le polymère est un polymère dérivé d'acide hyaluronique. Par « dérivé d'acide hyaluronique », on entend que le polymère peut être préparé à partir d'acide hyaluronique. En l'occurrence, les groupements de formules (I), (II) et (III) peuvent être obtenus par réticulation d'unités disaccharidiques d'acide hyaluronique ayant été préalablement oxydées. Un procédé de préparation du polymère dérivé d'acide hyaluronique est décrit ci-dessous.

Généralement, la totalité des unités disaccharidiques du polymère dérivé d'acide hyaluronique ne sont pas réticulées. Il comprend alors, en plus des groupements de formule (I), (II) et/ou (III), des unités de formule (IV) :

[Chem 4]

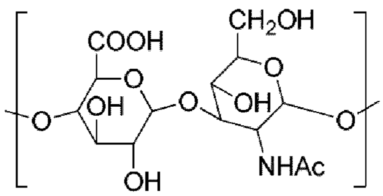
(IV)

chacune des unités de formule (IV) pouvant être sous forme neutre ou de sel.

Dans un mode de réalisation, la composition polymérique peut comprendre (voire consister en) le polymère dérivé d'acide hyaluronique sous forme sèche.

Dans un mode de réalisation, la composition polymérique comprend un solvant (voire consiste en le polymère dérivé d'acide hyaluronique dans un ou plusieurs solvants). Lorsque le solvant est ou comprend un milieu aqueux, notamment une solution aqueuse, telle que de l'eau, une solution tampon (par exemple une solution aqueuse de NaCl, notamment à 0,9% w/v) ou une solution physiologique, de préférence une solution physiologique, la composition polymérique se présente sous forme d'hydrogel. L'invention concerne également un hydrogel comprenant (voire constitué du) le polymère défini ci-dessus et un milieu aqueux.

Un hydrogel est un gel comprenant de l'eau. Un gel se caractérise par des mesures rhéologiques en mode dynamique et est défini par le fait que module de stockage (G') est supérieur au module de perte (G") sur toutes les fréquences de 0,01 à 10 Hz mesurées à 20°C.

L'hydrogel est transparent dans le domaine visible (de 400 à 800 nm), la transparence étant mesurée par un spectrophomomètre UV-visible, par exemple avec un appareil Varian modèle Cary 300 Scan, avec une mesure par exemple sur une couche de gel d'épaisseur de 10 mm.

L'hydrogel peut avantageusement être mis en forme, notamment par moulage, par extrusion ou par injection. Il a avantageusement la capacité de rester dans la forme qui lui a été donnée. Cette capacité s'explique notamment par le fait qu'il est réticulé et par sa rigidité.

L'hydrogel a une rigidité du même ordre de grandeur que le cerveau. Généralement, son module d'élasticité G' est compris de 200 Pa à 20 000 Pa, plus préférentiellement de 500 Pa à 15 000 Pa, et encore préféré de 1000 à 12000 Pa tel que mesuré par rhéomètre sous cisaillement à 20°C, par exemple un rhéomètre rotationnel Kinexus (Malvern, Les Ulis, France).

Dans un mode de réalisation, la composition polymérique est exempte d'agent anticancéreux, voire même d'agent thérapeutique.

Dans un autre mode de réalisation, la composition polymérique comprend un agent thérapeutique, de préférence un agent anti-inflammatoire, par exemple du kétorolac, et/ou une protéine.

Selon un deuxième objet, l'invention concerne un procédé de préparation d'un polymère dérivé d'acide hyaluronique (notamment celui défini ci-dessus) comprenant:

a) la fourniture d'un acide hyaluronique oxydé obtenu par oxydation d'acide hyaluronique par du périodate de sodium ($NaIO_4$),

b) la réaction de l'acide hyaluronique oxydé avec un agent de réticulation (« crosslinker » en anglais) de formule (XX) suivante :

$H_2N-O-L-O-NH_2$ (XX),

dans laquelle L est une chaîne hydrocarbonée comprenant de 2 à 10 atomes de carbone et éventuellement interrompue par un ou plusieurs groupes -O- ou -NH-.

Le procédé peut comprendre, avant l'étape a), une étape a0) d'oxydation d'acide hyaluronique par du périodate de sodium pour obtenir l'acide hyaluronique oxydé. Cette étape conduit à fournir l'acide hyaluronique oxydé.

De préférence, l'acide hyaluronique utilisé pour l'étape a0) a une masse moléculaire en poids (Mw) inférieure à 200 000 g/mol, notamment de 30 à 150 000 g/mol, de préférence de 45 à 120 000 g/mol, par exemple de 45 à 100 000 g/mol. La masse moléculaire peut être déterminée par analyse par exclusion chromatographique. Un acide hyaluronique ayant une telle masse moléculaire est moins visqueux et plus facile à manipuler qu'un acide hyaluronique de masse moléculaire plus élevée. Le gel comprenant le polymère obtenu est généralement plus cohésif et plus rigide que le gel obtenu à partir d'un acide hyaluronique de masse moléculaire plus élevé. De plus, un acide hyaluronique ayant une telle masse moléculaire conduit à un polymère dérivé d'acide hyaluronique qui déclenche généralement moins d'angiogenèse et/ou qui limite plus l'activation de chemins de signalisation cellulaires pro-cancéreux (voie CD44 ou voie PI 3-kinase / AKT) qu'un polymère obtenu à partir d'un acide hyaluronique de masse moléculaire plus élevée.

L'étape a0) consiste à oxyder de l'acide hyaluronique par du périodate de sodium.

Les groupes hydroxyles vicinaux (appelés diol) en position 2 et 3 du sucre acide D-glucuronique d'au moins une partie des unités disaccharides de l'acide hyaluronique subissent une coupure C-C oxydative en présence du périodate de sodium pour conduire à la formation de deux fonctions aldéhydes en position 2 et 3, comme illustré au schéma 1. Cette réaction d'oxydation est spécifique aux diols vicinaux.

[Chem 5]

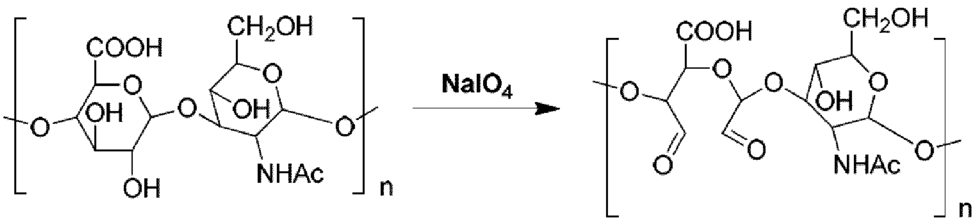


n représente le nombre d'unités disaccharide dans l'acide hyaluronique

Schéma 1 : Schéma réactionnel de l'oxydation de l'étape a0) sur une unité disaccharidique de l'acide hyaluronique.

L'acide hyaluronique oxydé fourni à l'étape a) et/ou obtenu à la fin de l'étape a0) comprend donc des unités de formule (X) suivante :

[Chem 6]

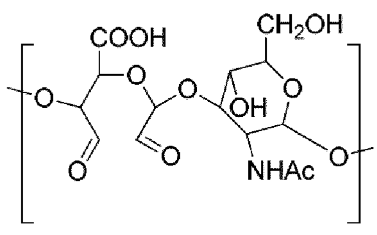
(X),

chacune des unités de formule (X) pouvant être sous forme neutre ou de sel.

Lors de l'étape a0), au moins une partie des unités disaccharides de l'acide hyaluronique est oxydée. Généralement, seules certaines unités disaccharides sont oxydées. L'acide hyaluronique oxydé fourni à l'étape a) et/ou obtenu à la fin de l'étape a0) comprend donc généralement des unités de formule (X) telle que définie ci-dessus et des unités de formule (IV) telle que définie ci-dessus.

L'étape a0) est de préférence réalisée à une température comprise de 0 à 15°C, par exemple de 1 à 10°C, en particulier de 2 à 5°C. Il est préférable de réaliser l'étape a0) à une température inférieure à la température ambiante (20°C) afin de limiter la dégradation de l'acide hyaluronique. De plus, les hydrogels préparés avec un acide hyaluronique

oxydé à ces températures sont plus rigides mécaniquement que les hydrogels obtenus avec un acide hyaluronique oxydé à température ambiante.

Le NaIO4 utilisé à l'étape a0) peut être supporté sur support solide, par exemple sur de la silice, ou non supporté.

Le procédé peut comprendre, entre les étapes a) et b), une étape a2) de lyophilisation de l'acide hyaluronique oxydé fourni à l'étape a) et/ou obtenu à la fin de l'étape a0). Cette lyophilisation permet de stocker sous forme stabilisée l'acide hyaluronique oxydé avant de l'utiliser dans l'étape b) qui suit. De préférence, le procédé comprend alors une étape a3) d'ajout d'un milieu aqueux à l'acide hyaluronique oxydé lyophilisé avant de réaliser l'étape b). Cette étape a3) permet que l'agent de réticulation se répartisse mieux au sein de l'acide hyaluronique oxydé, ce qui favorise la réaction de l'étape b).

Le milieu aqueux est notamment une solution aqueuse, telle que de l'eau, une solution tampon (par exemple une solution aqueuse de NaCl, notamment à 0,9% w/v) ou une solution physiologique, de préférence une solution physiologique.

L'étape b) de réaction de l'acide hyaluronique oxydé avec un agent de réticulation de formule (XX) permet de réticuler les chaines d'acide hyaluronique entre elles.

Dans la formule (XX), L est de préférence tel que défini ci-dessus.

L'agent de réticulation de formule (XX) comprend deux groupes terminaux oxyamine ($-O-NH_2$), qui réagissent avec les fonctions aldéhydes de l'acide hyaluronique oxydé par formation irréversible de liaisons oxime.

La réaction de couplage entre les fonctions aldéhydes et oxyamine est avantageusement bio-orthogonale, les molécules biologiques ne possédant pas naturellement de telles fonctions chimiques. Certaines molécules biologiques sont porteuses de fonctions amine, mais la réaction aldéhyde/amine est réversible et très lente par rapport à la réaction aldéhyde/oxyamine, et donc ne se fera pas en présence d'oxyamine.

L'étape b) est susceptible de conduire à la formation d'unité disaccharidique sous forme cyclique, de formule (XI) suivante, ou sous forme ouverte, de formule (XII) suivante :

[Chem 7]

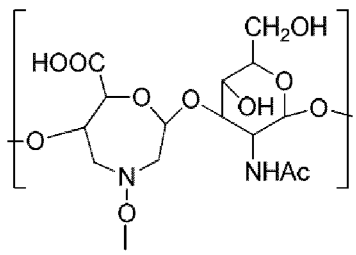

(XI),

[Chem 8]

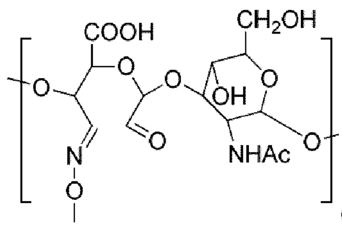

(XII)

où le groupe -N-O- est lié à une autre chaine d'acide hyaluronique par l'intermédiaire du groupe L, afin de former, avec une autre unité disaccharidique, les groupements de formule (I), (II) et/ou (III) tels que définis ci-dessus.

Le polymère dérivé d'acide hyaluronique obtenu à la fin de l'étape b) comprend ainsi des groupements de formules (I), (II) et/ou (III) telles que définis ci-dessus, sous forme neutre ou de sel.

Les exemples d'hydrogels à base de polymères dérivés d'acide hyaluronique préparés par réaction d'acide hyaluronique oxydé (tel qu'obtenu à la fin de l'étape a0) et/ou tel que fourni à l'étape a)) avec des agents de réticulation comprenant des groupes terminaux $-NH-NH_2$ (hydrazide) avaient des propriétés mécaniques qui se dégradaient dans le temps, inconvénient qui n'a pas été observé avec les hydrogels à base de polymère dérivé d'acide hyaluronique obtenu à partir de agents de réticulation de formule (XX) comprenant deux groupes terminaux $-O-NH_2$.

De préférence, lors de l'étape b), le ratio entre le nombre de moles d'agent de réticulation de formule (XX) et le nombre d'unités disaccharidiques de l'acide hyaluronique oxydé est compris de 0,01/1 à 1/1, notamment de 0,1/1 à

1/1, de préférence de 0,2/1 à 1/1, un ratio de 0,3/1 à 0,5/1 étant particulièrement préféré. Lorsque le ratio est supérieur à 1/1, l'agent de réticulation est mis en excès et donc gâché. Généralement, plus ce ratio est faible, plus l'hydrogel obtenu a un taux de gonflement élevé.

L'étape b) de réticulation est très rapide, typiquement de 10 secondes à une heure.

Le procédé comprend de préférence une étape de purification pour éliminer le $NaIO_4$ :

- soit entre les étapes a) et b) (étape a1) de purification de l'acide hyaluronique oxydé pour éliminer le $NaIO_4$),
- soit après l'étape b) (étape c) de purification du polymère dérivé d'acide hyaluronique pour éliminer le $NaIO_4$).

Cette étape permet d'éliminer le $NaIO_4$ cytotoxique. Elle peut par exemple être mise en oeuvre par ajout d'un alcool, tel que l'éthylène glycol, éventuellement suivi d'une dialyse, notamment une dialyse contre l'eau. Lorsque le procédé comprend à la fois une étape a1) de purification de l'acide hyaluronique oxydé et une étape a2) de lyophilisation de l'acide hyaluronique oxydé fourni à l'étape a) et/ou obtenu à la fin de l'étape a0), l'étape a2) est de préférence réalisée après l'étape a1). Le procédé comprend alors les étapes a), a1), a2) et b), dans cet ordre.

Les étapes a0) et b) sont généralement mises en oeuvre dans un milieu aqueux, notamment tel que défini ci-dessus. Lors de l'étape b), la proportion volumique de l'acide hyaluronique oxydé dans le milieu aqueux est de préférence supérieure ou égale à 5% w/v (en poids sur volume), ce qui favorise la réticulation. A la fin de l'étape b), le polymère dérivé d'acide hyaluronique se présente sous la forme d'un hydrogel.

Lors de l'étape b), l'hydrogel qui se forme prend typiquement la forme du récipient utilisé pour réaliser l'étape b). Par exemple, la mise en oeuvre de l'étape b) dans un récipient cylindrique permet de préparer un hydrogel sous forme cylindrique ou de pastille.

Le procédé peut comprendre une étape d) de mise en forme de l'hydrogel, par exemple par extrusion.

Afin d'obtenir le polymère dérivé d'acide hyaluronique sous forme sèche, le procédé peut comprendre une étape e) de séchage du polymère dérivé d'acide hyaluronique obtenu.

Selon un troisième objet, l'invention concerne le polymère dérivé d'acide hyaluronique susceptible d'être obtenu par le procédé ci-dessus. L'invention concerne également l'hydrogel susceptible d'être obtenu par le procédé ci-dessus lorsque l'étape b) est mise en oeuvre en milieu aqueux.

Selon un quatrième objet, l'invention concerne un kit comprenant :

- un premier compartiment comprenant un acide hyaluronique oxydé obtenu par oxydation d'acide hyaluronique par du périodate de sodium,
- un deuxième compartiment comprenant un agent de réticulation de formule (XX) telle que définie ci-dessus,

le kit se présentant de préférence sous forme d'une seringue d'injection, par exemple sous forme de seringue d'extrusion.

Le premier compartiment peut également comprendre un ou plusieurs agent(s) thérapeutique(s), de préférence un ou plusieurs agent(s) anti-inflammatoire(s).

Le kit selon l'invention permet avantageusement de préparer la composition polymérique selon l'invention, de préférence juste avant de l'utiliser.

Le kit se présente notamment sous forme d'une seringue d'injection 1 munie d'un piston 2 et d'une ouverture 3 sur laquelle une aiguille peut être fixée, l'ouverture 3 étant obturée par un premier opercule 4, ladite seringue d'injection 1 comprenant un premier compartiment 6 et un deuxième compartiment 7 séparés par un deuxième opercule 5 capable de se rompre lorsqu'une force est exercée sur le piston 2, où

- le premier compartiment 6 est localisé entre le premier opercule 4 et le deuxième opercule 5 et comprend un acide hyaluronique oxydé obtenu par oxydation d'acide hyaluronique par du périodate de sodium et éventuellement un ou plusieurs agent(s) thérapeutique(s),
- le deuxième compartiment 7 est localisé entre le deuxième opercule 5 et le piston 2 et comprend un agent de réticulation de formule (XX) telle que définie ci-dessus.

Un telle seringue d'injection est illustrée [fig 1] à la figure 1.

Le deuxième opercule 5 permet d'éviter que l'acide hyaluronique oxydé présent dans le premier compartiment 6 et l'agent de réticulation de formule (XX) présent dans le deuxième compartiment 7 ne soient en contact tant qu'aucune force n'est exercée sur le piston 2 (ou que la force est insuffisante pour le rompre).

Lorsqu'une force suffisante est exercée sur le piston 2, le deuxième opercule 5 localisé entre deuxième compartiment 7 et le premier compartiment 6 se rompt, ce qui conduit à la mise en contact de l'acide hyaluronique oxydé présent dans le premier compartiment 6 et de l'agent de réticulation de formule (XX) présent dans le deuxième compartiment 7, conduisant ainsi à leur réaction et donc à la mise en oeuvre du procédé défini ci-dessus. La composition polymérique selon l'invention se forme au sein de la seringue et est prête à être injectée.

Le premier opercule 4 permet d'éviter le contact entre l'environnement extérieur à la seringue et l'intérieur de la seringue. Typiquement, ce premier opercule 4 est ôté juste avant d'adapter une aiguille sur la seringue et d'injecter la composition polymérique.

L'acide hyaluronique oxydé présent dans le premier compartiment 6 et l'agent de réticulation de formule (XX) présent dans le deuxième compartiment 7 sont de préférence sous formes liquides. Par exemple, le premier compartiment 6 comprend l'acide hyaluronique oxydé dans un milieu aqueux, et/ou le deuxième compartiment 7 comprend l'agent de réticulation de formule (XX) dans un milieu aqueux, notamment tel que défini ci-dessus. Ceci facilite le mélange de l'acide hyaluronique oxydé et de l'agent de réticulation de formule (XX) lorsque le deuxième opercule 5 est rompu et une bonne homogénisation.

Il est possible que l'acide hyaluronique oxydé soit sous forme sèche, de préférence sous forme lyophilisée, dans le premier compartiment qui le contient dans le kit défini ci-dessus. Typiquement, le kit se présente alors sous forme d'une seringue d'injection munie :

- d'une ouverture 13 sur laquelle une aiguille peut être fixée, l'ouverture 13 étant obturée par un premier opercule 14,
- d'un premier piston 12 et d'un deuxième piston 11,
- d'un premier compartiment 16 comprenant un acide hyaluronique oxydé et lyophilisé obtenu par oxydation d'acide hyaluronique par du périodate de sodium puis lyophilisation, et éventuellement un ou plusieurs agent(s) thérapeutique(s), de préférence un ou plusieurs agent(s) anti-inflammatoire(s),
- d'un deuxième compartiment 17 comprenant un agent de réticulation de formule (XX) telle que définie ci-dessus,
- d'un troisième compartiment 18 comprenant un milieu aqueux,
- le premier compartiment 16 et le deuxième compartiment 17 étant séparés par un deuxième opercule 15 capable de se rompre lorsqu'une force est exercée sur le premier piston 12,
- le premier compartiment 16 et le troisième compartiment 18 étant séparés par un troisième opercule 19 capable de se rompre lorsqu'une force est exercée sur le deuxième piston 11,
- le deuxième compartiment 17 et le troisième compartiment 18 étant séparés par une paroi 20.

Un telle seringue d'injection est illustrée [fig 2] à la figure 2.

La paroi 20 permet d'éviter la mise en contact entre le milieu aqueux du troisième compartiment 18 et l'agent de réticulation de formule (XX) du deuxième compartiment 17. La paroi 20 est de préférence une paroi inamovible.

Le deuxième opercule 15 permet d'éviter que l'acide hyaluronique oxydé et lyophilisé présent dans le premier compartiment 16 et l'agent de réticulation de formule (XX) présent dans le deuxième compartiment 17 ne soient en contact tant qu'aucune force n'est exercée sur le piston 12 (ou que la force est insuffisante pour le rompre).

Le troisième opercule 19 permet d'éviter que l'acide hyaluronique oxydé et lyophilisé présent dans le premier compartiment 16 et le milieu aqueux présent dans le troisième compartiment 18 ne soient en contact tant qu'aucune force n'est exercée sur le piston 11 (ou que la force est insuffisante pour le rompre).

Le premier piston 12 et le deuxième piston 11 peuvent être actionnés indépendamment l'un de l'autre.

Typiquement, pour utiliser la seringue d'injection, on applique une force suffisante sur le piston 11 de manière à rompre le troisième opercule 19, puis une force suffisante sur le piston 12 de manière à rompre le deuxième opercule 15.

Lorsqu'une force suffisante est exercée sur le piston 11, le troisième opercule 19 localisé entre troisième compartiment 18 et le premier compartiment 16 se rompt, ce qui conduit à la mise en contact de l'acide hyaluronique oxydé et lyophilisé présent dans le premier compartiment 6 et du milieu aqueux présent dans le troisième compartiment 18, conduisant ainsi à l'hydratation de l'acide hyaluronique oxydé et lyophilisé et à la formation d'un milieu aqueux comprenant l'acide hyaluronique oxydé.

Ensuite, lorsqu'une force suffisante est exercée sur le piston 12, le deuxième opercule 15 localisé entre deuxième compartiment 7 et le premier compartiment 6 se rompt, ce qui conduit à la mise en contact entre le milieu aqueux comprenant l'acide hyaluronique oxydé et l'agent de réticulation de formule (XX) présent dans le deuxième compartiment 17, conduisant ainsi à leur réaction et donc à la mise en oeuvre du procédé défini ci-dessus. La composition polymérique selon l'invention se forme au sein de la seringue et est prête à être injectée.

Le premier opercule 14 permet d'éviter le contact entre l'environnement extérieur à la seringue et l'intérieur de la seringue. Typiquement, ce premier opercule 14 est ôté juste avant d'adapter une aiguille sur la seringue et d'injecter la composition polymérique.

Quel que soit le mode de réalisation considéré pour le kit, celui-ci est de préférence mis sous agitation afin que les milieux mis en contact se mélangent bien.

Par exemple, juste avant exérèse de la tumeur, une force est exercée sur le piston 11 de manière que le troisième opercule 19 se rompe. Pendant le temps d'intervention d'exérèse, la seringue est mise sous agitation, afin de favoriser la formation d'un milieu aqueux comprenant l'acide hyaluronique oxydé homogène. A la fin de l'exérèse, une force est exercée sur le piston 12 de manière que le deuxième opercule 19 se rompe et que la composition polymérique se forme, et la seringue est mise sous agitation. Ensuite, le premier opercule est ôté afin de permettre l'injection de la

composition polymérique dans la cavité d'exérèse, typiquement en exerçant une force à la fois sur le premier piston 12 et le deuxième piston 11.

Selon un cinquième objet, l'invention concerne l'hydrogel défini ci-dessus pour son utilisation pour limiter la récidive d'une tumeur, de préférence du glioblastome, du cancer du sein, du pancréas, de la vessie, ou du sarcome chez un patient ayant subi une intervention chirurgicale d'exérèse de la tumeur.

La tumeur est généralement une tumeur cancéreuse.

L'hydrogel selon l'invention, une fois introduit dans la cavité d'exérèse de la tumeur, permet avantageusement de limiter l'inflammation locale post-opératoire et de limiter la récidive de la tumeur. Sans vouloir être liés par des théories particulières, les inventeurs supposent que cette réduction de l'inflammation s'expliquerait d'une part par la propriété antiinflammatoire intrinsèque de l'hydrogel, et d'autre part grâce à la capacité de l'hydrogel à combler la cavité d'exérèse, cette capacité s'expliquant notamment grâce à ses propriétés mécaniques, en particulier sa rigidité, proches de celle du cerveau, qui lui permettent de combler la cavité d'exérèse de façon cohésive. Avantageusement, l'hydrogel permet de limiter l'inflammation plus de trois mois. Un délai de 2 semaines post-chirurgie permet de prendre en charge les phases d'inflammation aigues, et de 3 mois permet de traiter l'inflammation aigue et l'inflammation chronique.

L'hydrogel est avantageusement biocompatible, et ce sur au moins trois mois.

Généralement, l'hydrogel est toujours présent après 3 mois d'implantation dans ladite cavité. Il se dégrade donc peu, voire pas, *in vivo.*

Généralement, la viabilité cellulaire au sein de l'hydrogel telle que déterminée selon la norme ISO 10993_5 est supérieure à 70% à 29 jours.

Selon un sixième objet, l'invention concerne l'hydrogel défini ci-dessus pour son utilisation comme produit de comblement (« filler » en anglais) d'une cavité d'exérèse d'une tumeur, de préférence du glioblastome, du cancer du sein, du pancréas, de la vessie, ou du sarcome.

L'invention concerne également une méthode de traitement et/ou de prévention de la récidive d'une tumeur comprenant :

- l'exérèse de la tumeur d'un patient en ayant besoin, ce par quoi une cavité d'exérèse est formée, puis
- le comblement de la cavité d'exérèse par l'hydrogel défini ci-dessus.

Le comblement peut être réalisé par injection de l'hydrogel dans la cavité d'exérèse.

Selon un septième objet, l'invention concerne l'hydrogel défini ci-dessus pour son utilisation pour le traitement d'une inflammation.

L'invention concerne également une méthode de traitement de l'inflammation d'un patient en ayant besoin comprenant l'administration par injection de l'hydrogel tel que défini ci-dessus.

Les exemples et figures qui suivent illustrent l'invention.

[Fig 1] La figure 1 est un schéma en coupe d'une seringue d'injection 1 selon l'invention et telle que définie ci-dessus, la seringue étant munie d'un piston 2 et d'une ouverture 3, l'ouverture 3 étant obturée par un premier opercule 4, ladite seringue d'injection 1 comprenant un premier compartiment 6 et un deuxième compartiment 7 séparés par un deuxième opercule 5 capable de se rompre lorsqu'une force est exercée sur le piston 2.

[Fig 2] La figure 2 est un schéma en coupe d'une seringue d'injection 10 selon l'invention et telle que définie ci-dessus, la seringue étant munie :

- d'une ouverture 13 sur laquelle une aiguille peut être fixée, l'ouverture 13 étant obturée par un premier opercule 14,
- d'un premier piston 12,
- d'un deuxième piston 11,
- d'un premier compartiment 16,
- d'un deuxième compartiment 17,
- d'un troisième compartiment 18,
- le premier compartiment 16 et le deuxième compartiment 17 étant séparés par un deuxième opercule 15 capable de se rompre lorsqu'une force est exercée sur le premier piston 12,
- le premier compartiment 16 et le troisième compartiment 18 étant séparés par un troisième opercule 19 capable de se rompre lorsqu'une force est exercée sur le deuxième piston 11,
- le deuxième compartiment 17 et le troisième compartiment 18 étant séparés par une paroi 20.

Dans les exemples ci-dessous, les termes « gel » et « hydrogel » ont la même signification. Par exemple, l'hydrogel G1 correspond au gel G1.

Exemple 1 : préparation de polymères et des hydrogels

Les polymères HA utilisés comme produits de départ sont fournis par la société Contipro (République Tchèque) :

- HyActive de poids moléculaire 50-80 kDa, noté « low HA » ci-dessous,
- HySilk de poids moléculaire 250-450 kDa, noté « high HA » ci-dessous.

Les polymères sont préparés en deux étapes a0) et b) et une purification ou lavage, comme illustré au schéma 2.

[Chem 9]

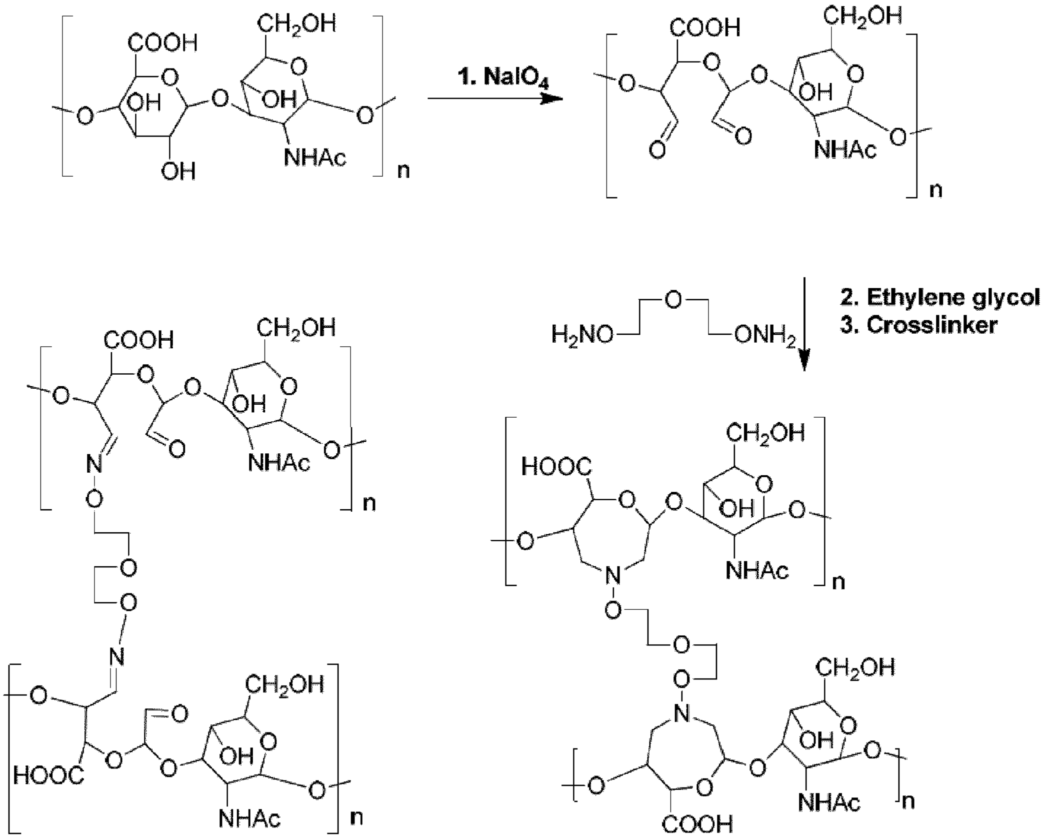


Schéma 2 : Schéma réactionnel de préparation de polymères à partir d'acide hyaluronique et d'un agent de réticulation de formule $H_2N-O-(CH_2)_2-O-(CH_2)_2-O-NH_2$.

L'étape a0) d'oxydation d'acide hyaluronique par du périodate de sodium ($NaIO_4$) permet d'obtenir un acide hyaluronique oxydé, noté « HAox » ci-dessous.

Exemple 1.1 : étape a0) d'oxydation du HA par $NaIO_4$ en milieu homogène à température ambiante (20°C)

A la solution aqueuse de HA (HyActive 100 mg/mL ou HySilk 50 mg/mL) est ajouté le $NaIO_4$ en proportions indiquées dans le Tableau 1, et le milieu réactionnel est agité durant une nuit à température ambiante. Les résidus de $NaIO_4$ n'ayant pas réagi sont alors inactivés par l'éthylène glycol (0.2 équivalent molaire par rapport à $NaIO_4$) rajouté au milieu réactionnel et incubé pendant 1h à température ambiante. Une solution de HA oxydé est alors obtenue (notée « HAox-Tamb »).

[Tableau 1]

Tableau 1. Quantités introduites pour l'oxydation de HA

| Gel | Concentration HA | Concentration $NaIO_4$ |
|---|---|---|
| High HA 5% 1:1* (HySilk) | 50 mg/mL | 85 mM |
| Low HA 10% 1:1* (HyActive) | 100 mg/mL | 170 mM |
| Low HA 10% 1:0.5** (HyActive) | 100 mg/mL | 85 mM |
| * : 1 équivalent de $NaIO_4$ par unité disaccharidique du HA<br>** : 0,5 équivalent de $NaIO_4$ par unité disaccharidique du HA | | |

Exemple 1.2 : oxydation du HA par $NaIO_4$ en milieu homogène à 4°C

A la solution aqueuse de HA (HyActive 100 mg/mL ou HySilk 50 mg/mL) est ajouté le $NaIO_4$ en proportions indiquées dans le Tableau 1, et le milieu réactionnel est agité durant une nuit à 4°C. Les résidus de $NaIO_4$ n'ayant pas réagi sont alors inactivés par l'éthylène glycol (0.2 équivalent molaire par rapport à $NaIO_4$) rajouté au milieu réactionnel et incubé pendant 1h à température ambiante. Une solution de HA oxydé est alors obtenue (notée « HAox-4° »).

Exemple 1.3 : analyse SEC du HA, HAox-Tamb et HAox-4°

Les solutions de polymère HA, HAox-Tamb, et HAox-4° sont analysées par chromatographie d'exclusion stérique (SEC, « Size Exclusion Chromatography » en anglais).

La chromatographie d'exclusion a été réalisée sur un instrument HPLC shimadzu équipé d'une colonne d'exclusion SuperMultipore TOSOH TSK gel, d'une colonne de garde du même matériau, et d'une boucle d'échantillon de 100 µL. Les élutions de pics ont été contrôlées en ligne avec un détecteur d'absorbance UV-VIS, un détecteur de diffusion de lumière multi-angle (MALS) Wyatt Dawn Helios et un détecteur d'indice de réfraction différentiel (dRI) Wyatt Optilab rEX. Le tampon d'élution pour HPLC était constitué de 0,1 M de NaNOs + 0,01 M de PB (tampon phosphate sans solution saline) à pH 7. Le débit a été fixé à 0,6 mL / min. Toutes les mesures ont été effectuées à 30 ° C. Pour la mesure, les échantillons d'HA (Low HA, High HA) ont été dissous à 10 mg / mL dans le tampon d'élution en le maintenant sous agitation magnétique à température ambiante jusqu'à obtention d'une solution limpide. Les échantillons HAox ont été préparés comme décrit dans l'exemple 1.1 (HAox-Tamb) et l'exemple 1.2 (HAox-4°). Trois injections répétées ont été effectuées à la concentration finale de 1 mg / mL (High HA, oxydé ou non) ou à 5 mg / mL (Low HA, oxydé ou non), après dilution des échantillons avec le tampon d'élution pour obtenir la concentration souhaitée pour l'analyse. Le dndc utilisé pour calculer le poids moléculaire a été mesuré en mode « batch » (logiciel Astra), en réalisant une courbe d'étalonnage à 5 points dans la plage de 0,1 à 1 mg / mL. Trois répétitions ont été effectuées, obtenant une valeur de dndc de 0,1426 mL / g (Low HA) ou 0,1423 mL / g (High HA). Les valeurs de Mw, Mn et PdI et les distributions de poids cumulés ont été calculées à l'aide du logiciel Astra 6.1.

Les résultats obtenus sont regroupés Tableau 2 pour le low HA et Tableau 3 pour le High HA.

[Tableau 2]

Tableau 2. Résultats de l'analyse SEC pour le Low-HA.

| | Mn (g/mol) | Mp (g/mol) | Mw (g/mol) | Mz (g/mol) | Mw/Mn | Mz/Mn |
|---|---|---|---|---|---|---|
| HA Low (produit de départ) | 4,4 (0,2) $10^4$ | 4,5 (0,1) $10^4$ | 5,50 (0,04) $10^4$ | 6,88 (0,05) $10^4$ | 1,24 (0,03) | 1,55 (0,06) |
| HAox-Tamb (obtenu à l'expl 1.1.) | 3,0 (0,1) $10^4$ | 3,21 (0,09) $10^4$ | 3,8 (0,1) $10^4$ | 5,0 (0,2) $10^4$ | 1,273 (0,004) | 1,65 (0,07) |
| HAox-4° (obtenu à l'expl 1.2.) | 4,3 (0,2) $10^4$ | 4,6 (0,2) $10^4$ | 5,7 (0,4) $10^4$ | 8,8 (0,5) $10^4$ | 1,32 (0,02) | 2,045 (0,02) |

[Tableau 3]

Tableau 3. Résultats de l'analyse SEC pour le High-HA.

| | Mn (g/mol) | Mp (g/mol) | Mw (g/mol) | Mz (g/mol) | Mw/Mn | Mz/Mn |
|---|---|---|---|---|---|---|
| HA High (produit de départ) | 2,05 (0,02) $10^5$ | 2,26 (0,08) $10^5$ | 2,43 (0,01) $10^5$ | 2,85 (0,01) $10^5$ | 1,19 (0,01) | 1,39 (0,01) |
| HAox-Tamb (obtenu à l'expl 1.1.) | 5,6 (0,4) $10^4$ | 6,3 (0,4) $10^4$ | 7,1 (0,5) $10^4$ | 8,8 (0,5) $10^4$ | 1,25 (0,01) | 1,55 (0,01) |
| HAox-4° (obtenu à l'expl 1.2.) | 9,0 (0,7) $10^4$ | 1,0 (0,1) $10^5$ | 1,2 (0,1) $10^5$ | 1,5 (0,1) $10^5$ | 1,29 (0,05) | 1,63 (0,02) |

Nous observons que l'oxydation du HA conduite à 4°C préserve mieux la structure du polymère que celle conduite à température ambiante puisque les masses molaires du HA sont mieux préservées (notamment moins de 5% de perte de poids moléculaire pour le HAlow). La dégradation du polymère est également beaucoup moins importante pour le HA de faible poids moléculaire. Cela conduira à des gels réticulés présentant des propriétés mécaniques plus rigides (exemple 3.2).

Exemple 1.4 : oxydation du HA par $NaIO_4$ sur support solide à température ambiante

Synthèse des billes / $NaIO_4$ à 10% massique : le périodate de sodium (4 g) est solubilisé dans l'eau (100 mL) et la solution portée à 60°C. De la silice (40g / 230-400 mesh) est ensuite ajoutée au milieu et l'agitation maintenue à 60°C durant 1 heure. L'eau est finalement évaporée et le solide obtenu est mis à l'étuve durant 16 heures à 120°C.

Des billes de silice / $NaIO_4$ à 10% (276 mg / synthétisées au laboratoire comme décrit ci-dessus) sont mises en suspension dans une solution de low HA à 6,6% massique (1,533 mL) dans l'eau. Le milieu réactionnel est agité à température ambiante durant 2 heures puis filtré sur du sable et coton à travers une colonne. Le filtrat est récupéré sans aucune purification ultérieure en vue de l'étape de réticulation.

L'étape b) est la réaction du HAox avec un agent de réticulation bis(oxyamine) de formule $H_2N-O-(CH_2)_2-O-(CH_2)_2-O-NH_2$. (« L1 » ci-dessous) ou $H_2N-O-(CH_2)_2-O-(CH_2)_2-O-(CH_2)_2-O-NH_2$.

Exemple 1.5 : Synthèse de l'agent de réticulation bis(oxyamine) L1

L'agent de réticulation éthylène glycol bis-oxyamine est synthétisé au laboratoire suivant le schéma 3, en deux étapes avec un rendement de synthèse global de 65%.

[Chem 10]

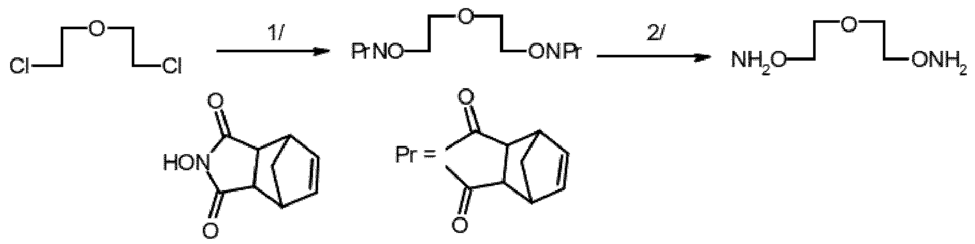


Schéma 3 : Schéma de synthèse du réticulant bis-oxyamine L1

L'hydroxylamine N-norbornène, groupe protecteur de la fonction oxyamine-ONH2, (1,8 g / 10 mmoles / 2,5 éq.) est dissout dans le DMF (22 mL), en présence de carbonate de potassium (1,4 g / 25 mmoles / 2,5 éq.). Le dichloro-éthylène glycol (470 µL / 4 mmoles) est ajouté à la solution et le milieu réactionnel porté à 50 °C sous agitation et laissé durant 24 heures. Le solvant est ensuite évaporé sous pression réduite, et le résidu obtenu (2g) est lavé par extraction dichlorométhane-eau, séché sur sulfate de sodium, filtré puis le solvant évaporé pour conduire à l'obtention d'un solide blanc.

Pour l'étape de la déprotection des fonctions oxyamine de l'agent de réticulation, le solide brut est mis en suspension dans l'éthanol absolu et de l'hydrazine y est rajoutée, l'agitation maintenue durant 2 heures au reflux. Tout comme la première étape de synthèse, la réaction est suivie par chromatographie sur couche mince et le produit cible formé (agent de réticulation oxyamine) est purifié par chromatographie sur gel de silice (éluant : dichlorométhane 10 / méthanol 1 : v/v) et obtenu à une quantité de 887 mg (rendement global de 65%). L'agent de réticulation est un liquide, de densité 1,25 g/mL.

Le produit est caractérisé par RMN 1H du proton (Bruker BioSpin GmbH) : RMN 1H (400 MHz, $CDCl_3$): $\delta$ = 3,65 - 3,7 (m, 4H, CH2-1, CH2-1'), 3,85 - 3,90 (m, 4H, CH2-2, CH2-2'), 5,57 (s, 4H, O-NH2).

Exemple 1.6 : Synthèse de l'agent de réticulation bis(oxyamine) L2

L'agent de réticulation di-éthylène glycol bis-oxyamine est synthétisé par anlogie, suivant le schéma 4, en deux étapes avec un rendement de synthèse global de 68%.

[Chem 11]

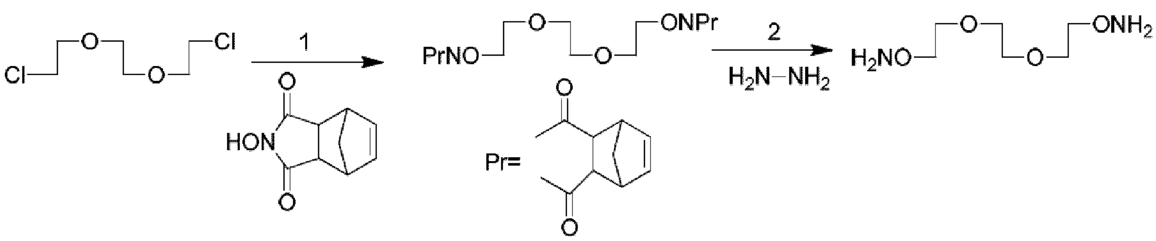


Schéma 4 : Schéma de synthèse du réticulant bis-oxyamine L2

L'hydroxylamine N-norbornène, groupe protecteur de la fonction oxyamine-ONH2, (9 g / 50 mmoles / 2,5 éq.) est dissout dans le DMF (110 mL), en présence de carbonate de potassium (7 g / 25 mmoles / 2,5 éq.). Le dichloro-di-éthylène glycol (3,125 mL / 20 mmoles) est ajouté à la solution et le milieu réactionnel porté à 50 °C sous agitation et laissé durant 24 heures. Le solvant est ensuite évaporé sous pression réduite, et le résidu obtenu (2g) est lavé par extraction acétate d'éthyle-eau, séché sur sulfate de sodium, filtré puis le solvant évaporé pour conduire à l'obtention d'un solide blanc.

Pour l'étape de la déprotection des fonctions oxyamine de l'agent de réticulation, le solide obtenu est mis en suspension dans l'éthanol absolu (110 mL) et de l'hydrazine (2,7 mL) y est rajoutée, l'agitation maintenue durant 2 heures au reflux. Tout comme la première étape de synthèse, la réaction est suivie par chromatographie sur couche mince et le produit cible formé (agent de réticulation oxyamine L2) est purifié par chromatographie sur gel de silice (éluant : dichlorométhane 10 / méthanol 1 : v/v) et obtenu à une quantité de 2,47g (rendement global de 68%) sous forme de liquide de densité 1,24 g/mL.

Le produit est caractérisé par RMN 1H du proton (Bruker BioSpin GmbH) : RMN 1H (400 MHz, $CDCl_3$): $\delta$ = 3,67 (s, 4H, O-CH2-CH2-O), 3,68 - 3,71 (m, 4H, CH2-1, CH2-1'), 3,83 - 3,87 (m, 4H, CH2-2, CH2-2'), 5,53 (s, 4H, O-NH2).

Exemple 1.7 : Préparation des gels et moulages de pastilles de gels, avec purification des gels par lavage après réticulation avec l'agent de réticulation L1

Le milieu réactionnel contenant le HA oxydé par $NaIO_4$ tel que préparé dans l'exemple 1.1 (température ambiante) ou l'exemple 1.2 (4°C) est coulé dans des moules en Téflon de 4, 6 ou 12 mm de diamètre pour réaliser l'étape de réticulation.

La réticulation du HAox est réalisée avec l'ajout au brut réactionnel de l'agent de réticulation bis(oxyamine) L1 à la concentration décrite dans le Tableau 4. La gélification se fait en quelques secondes.

Après 10 minutes à température ambiante (20°C), les gels sont démoulés et placés dans des plaques à puits où ils sont rincés à l'eau puis dans une solution de chlorure de sodium à 0.9%. Ils sont enfin stockés à 4°C.

[Tableau 4]

Tableau 4 : concentration en HAox et en agent de réticulation L1 pour former des gels mis en forme sous forme de pastille.

| | Gel | Concentration HAox | Concentration L1 |
|---|---|---|---|
| Gel G3 | High HA 5% 1:1* | 50 mg/mL | 0,35 % v/v |
| Gel G1 | Low HA 10% 1:1* | 100 mg/mL | 0,7 % v/v |
| Gel G2 | Low HA 10% 1:0.5** | 100 mg/mL | 0,35 % v/v |
| * : 1 équivalent de $NaIO_4$ et 0.43 équivalent d'agent de réticulation L1 par unité disaccharidique du HA<br>** : 0,5 équivalent de $NaIO_4$ et 0.22 équivalent d'agent de réticulation L1 par unité disaccharidique du HA | | | |

Exemple 1.8 : étape c) mise en oeuvre après l'étape b) : élimination du $NaIO_4$ du gel formé à l'exemple 1.1

Les composés pouvant présenter une éventuelle cytotoxicité sont principalement le périodate de sodium $NaIO_4$, l'éthylène glycol et le glycérol et des sous-produits formés lors de l'étape d'oxydation.
Des études de lavage à l'eau des gels formés ont été effectuées et suivies en HPLC

- HPLC (Waters analytique, Alliance)
- Colonne: Symmetry C18 / 3,5 μm / 4,6×75 mm
- Programme :
  - Débit: 1mL/min
  - Voie A: ACN / 0,1% TFA - voie B: Eau / 0,1% TFA
  - 5% A à 100% A en 20'
  - Détection à 320 nm et 270 nm

Les résultats obtenus montrent que 3 lavages successifs avec changement de bain, d'une durée d'une heure à deux heures chacun, sont nécessaires pour éliminer totalement les impuretés.

Les gels obtenus sont cohésifs et ne se redissolvent pas dans l'eau ou en tampon biologique, comme il sera montré à l'exemple 4.3.

Exemple 1.8 : Préparation des gels et moulages de pastilles de gels, avec purification des gels par lavage après réticulation avec l'agent de réticulation L2

Le milieu réactionnel contenant le HAlow 1:1 oxydé par $NaIO_4$ tel que préparé dans l'exemple 1.1 (température ambiante (20°C)) est coulé dans des moules en Téflon de 4, 6 ou 12 mm de diamètre pour réaliser l'étape de réticulation.

La réticulation du HAox est réalisée avec l'ajout au brut réactionnel de l'agent de réticulation bis(oxyamine) L2 au nombre d'équivalent de HA : agent de réticulation de 1 : 0,43. La gélification se fait en quelques secondes. Après 10 minutes à température ambiante (20°C), les gels sont démoulés et placés dans des plaques à puits où ils sont rincés à l'eau puis dans une solution de chlorure de sodium à 0.9%. Ils sont enfin stockés à 4°C.

Exemple 1.9 : Extrusion par une seringue 18G de l'hydrogel G1 de l'exemple 1.7 pour obtenir le gel G4 sous forme extrudée

Le gel G1 décrit dans l'exemple 1.7 préparé dans de petits moules de 4 mm de diamètre est introduit dans le barillet d'une seringue et extrudé avec une seringue de gauge 18G. Le gel extrudé est cohésif, et est noté « gel G4 ».

Exemple 1.10 : étape c) mise en oeuvre entre les étapes a) et b) : purification du $NaIO_4$ résiduel du HAox sur gel de silice, puis étape c) de réticulation des gels et mise en forme sous forme de pastilles

Etape c) : Le milieu réactionnel contenant le HA oxydé par $NaIO_4$ tel que préparé dans l'exemple 1.1 (température ambiante) ou l'exemple 1.2 (4°C) est purifié sur colonne de silice SiOH. La colonne contient du sable sur coton à sa base afin d'éviter que la silice séparatrice (couche d'1 cm) ne soit récupérée dans le filtrat.

Etape b) : Un volume de 500 μL du filtrat de la solution de HAox est récupéré et est mis dans un moule en Téflon de 1,1 cm de diamètre puis une solution d'agent de réticulation (40 μL de la solution mère à 10% massique dans l'eau) y est ajoutée. Après un bref mélange de la solution, le gel se forme au bout d'environ 30 secondes.

Exemple 1.11: réticulation des gels à partir de HAox préparé suivant l'exemple 1.4 (HA oxydé par $NaIO_4$ sur support solide) en pastilles

Etape b) : Un volume de 500 µL du filtrat de la solution de HAox est récupéré et est mis dans un moule en téflon de 1,1 cm de diamètre puis une solution d'agent de réticulation (20 µL de la solution mère à 10% massique dans l'eau) y est ajoutée. Après un bref mélange dans le moule, le gel se forme au bout d'environ 60 secondes.

Exemple 2 (comparatif) : Synthèse d'un gel hydrazone pas stable

Le milieu réactionnel contenant le HAlow 1 :1 oxydé par $NaIO_4$ tel que préparé dans l'exemple 1.1 (température ambiante) est placé dans un pilulier et la réticulation du HAox est réalisée avec l'ajout au brut réactionnel de l'agent de réticulation acide adipique hydrazide (AAH), ayant une fonction hydrazide $-NH-NH_2$ à chacune de ses extrémités, avec un nombre d'équivalent de HA : agent de réticulation de 1 : 0,43. La gélification se fait en quelques secondes.

Cependant, le gel obtenu n'est pas stable comparé au gel formé avec l'agent de réticulation L1, car après dix heures, le gel jaunit puis se dégrade en liquide.

De même, le gel obtenu avec l'agent de réticulation hydrazide AAH par moulage 12 mm est complètement dissout lors de l'étape de lavage puis de stockage, alors que les gels des exemples 1.7 et 1.8 restent en forme et ne se dissolvent pas/ ne se dégradent pas lors des lavages.

Exemple 3 : Caractérisation des hydrogels

Exemple 3.1 : caractérisation mécanique et de gonflement des hydrogels de l'exemple 1.7 préparés avec du HA oxydé à température ambiante

Les propriétés de gonflement des hydrogels de l'exemple 1.7 (préparés avec du HA oxydé à température ambiante) ont été caractérisés dans l'eau et le NaCl 0.9 % m/v.

Pour les mesures du taux de gonflement, les hydrogels gonflés à l'équilibre à température ambiante (c'est à dire dans le tampon d'intérêt depuis au moins 48 h) sont égouttés pendant 30 s sur papier filtre sur chaque face puis pesés (Ww, poids gonflé). Ils sont ensuite congelés à -20°C pendant une nuit puis lyophilisés pendant 24h. Le poids sec de polymère lyophilisé est ensuite mesuré (Wd, poids sec).

Le taux de gonflement (SR) est alors calculé selon l'équation suivante:

$$SR = (Ww - Wd) / Wd.$$

3 à 6 échantillons / point ont été mesurés (Tableau 5).

[Tableau 5]

| Polymère | SR dans l'eau | SR dans une solution aqueuse de NaCl 0,9% |
|---|---|---|
| High HA 5% 1:1 | 370 ± 100 | 35.1 ± 0.4 |
| Low HA 10% 1:1 (Gel G1) | 46.7 ± 9.9 | 15.7 ± 0.3 |
| Low HA 1 0% 1:0.5 (Gel G2) | 62.5 ± 5.3 | 17.6 ± 0.4 |

Tableau 5. Taux de gonflement à température ambiante (20°C) des hydrogels de l'exemple 1.7 (préparés avec du HA oxydé à température ambiante). Les hydrogels sont gonflés pendant 48h et il est vérifié que l'état d'équilibre de gonflement était atteint (c'est à dire pas de gain en poids supplémentaire au bout de 24h).

Les courbes contrainte-déformation (contrainte en kPa en fonction de la déformation en %) en compression des hydrogels de l'exemple 1.7 (préparés avec du HA oxydé à température ambiante) dans l'eau et le NaCl 0.9 % m/v sont ensuite mesurés à l'aide d'un texturomètre TAXT-Plus. On a observé que :

- le gonflement est plus important dans l'eau que dans la solution aqueuse de NaCl 0,9%;
- le gonflement est plus important lorsque la concentration en agent de réticulation est plus faible.
- les gels HA high sont très collants et élastiques. Ils ne tiennent pas bien en forme et ont tendance à s'étaler.
- les gels HA low sont plus durs et plus cassants que les gels high HA. Leur forme est conservée pendant plusieurs mois dans l'eau ou en tampon NaCl 0,9% w/v.

- les gels dans le NaCl sont plus élastiques et moins cassants que ceux dans l'eau.

Exemple 3.2 : caractérisation mécanique des hydrogels G1 de l'exemple 1.7 préparés avec du HA oxydé à température ambiante ou à 4°C

Les courbes contrainte-déformation en compression des hydrogels G1 de l'exemple 1.7 préparé avec du HA oxydé à température ambiante G1(Tamb) ou du HA oxydé à 4°C G1(4°C) et gonflés plus de 48 h dans NaCl 0.9 % m/v à température ambiante sont mesurés à l'aide d'un texturomètre TAXT-Plus.
Les valeurs des modules élastiques (G') et visqueux (G") des hydrogels G1 de l'exemple 1.7 préparé avec du HA oxydé à température ambiante G1(Tamb) ou du HA oxydé à 4°C G1(4°C) et gonflés dans NaCl 0.9 %m/v pendant au moins 48h à température ambiante sont mesurés à l'aide d'un rhéomètre rotationnel Kinexus (Malvern, Les Ulis, France) (mesure en cisaillement, entre 0.01 et 10 Hz).
Les matériaux obtenus sont bien des hydrogels élastiques, cohésifs et stables (G' et G" environ constants quelle que soit la fréquence, G' > G")
Les hydrogels préparés avec le HA oxydé à 4°C sont plus rigides mécaniquement que les hydrogels obtenus avec du HA oxydé à température ambiante. La température de l'étape a0) d'oxydation impacte donc les propriétés mécaniques du gel obtenu.

[Tableau 6]

| | G' (Pa) à 10 Hz |
|---|---|
| Gel G1(Tamb) | 9700 |
| Gel G1(4°C) | 3700 |

Exemple 3.3 : caractérisation mécanique du gel extrudé G4 de l'exemple 1.9

Les valeurs des modules élastiques (G') et visqueux (G") de l'hydrogel G4 de l'exemple 1.9 préparé avec du HA oxydé à température ambiante sont mesurés à l'aide d'un rhéomètre rotationnel Kinexus (Malvern, Les Ulis, France) (mesure en cisaillement, de 0,1 à 100 Hz).
L'extrusion par une aiguille de gauge 18G ne modifie pas les propriétés de l'hydrogel (G' = 3 700 Pa pour l'hydrogel G1(HAox Tamb) avant extrusion, versus G' = 4 300 Pa (à 10 Hz) pour l'hydrogel G4 après extrusion.

Exemple 4 : Tests *in vitro* des hydrogels

Exemple 4.1 : test de cytotoxicité

Le test de cytotoxicité est effectué selon la norme ISO 10993_5 sur 29 jours.
Pour cela, nous faisons des tests, à partir des extraits des hydrogels, sur cellules L929 (fibroblaste de souris). Au cours de ce test, nous étudions aussi la dégradation, dans le temps, des hydrogels en milieu biologique et la stérilité.
Le protocole est le suivant :

Jour 1 :

- Incubation de l'hydrogel à tester dans du milieu de culture cellulaire, EMEM sans rouge phénol + 10% FBS (foetal bovine sérum) + 1% Glutamax, aussi appelé milieu d'extraction (3 $cm^2$/ml) pendant 29 jours à 37°C sous agitation (80 rpm).

Jour X :

- Les milieux d'extraction sont récupérés à jour 1 puis tous les 3 à 4 jours (Fig 11) et congelés à -20°C en attente du test de cytotoxicité.

Jour 29 :

- Ensemencement des cellules (lignée cellulaire L929) dans des plaques 96 puits, 10 000 cellules/puit.

Dépôt de 100 µl/puit de milieu de culture EMEM + 10% FBS + 1% Glutamax.

Jour 30 :

- Aspiration du milieu de culture (EMEM + 10% FBS + 1% Glutamax) déposé le jour 29 pendant l'ensemencement.
- Décongélation des milieux milieu d'extraction (EMEM sans rouge phénol + 10% FBS + 1% Glutamax)

récupérés à jour 1 puis tous les 3 à 4 jours.

- Dépôt des milieux d'extraction sur cellules pour 24h d'incubation à 37°C, 5% CO2.

Jour 31 :

- L'évaluation de la viabilité cellulaire se fait via un test MTS-PMS sur les cellules incubées avec les milieux d'extraction. Ce test de viabilité utilise une méthode colorimétrique pour la quantification sensible des cellules viables. En effet, le MTS-PMS change de couleur lorsqu'il se lie à un déchet métabolique de la cellule vivante.

Dépôt du MTS-PMS, 20 μl/puits.

- La lecture de l'absorbance se fait sur un lecteur de plaque (FLUOstar Omega) et le traitement des données se fait par analyse Excel.
- Au-dessus de 70% de viabilité cellulaire (comparaison au blanc : cellules + milieu de culture), les échantillons testés ne sont pas cytotoxiques, en dessous l'hydrogel testé est cytotoxique.

Deux paramètres nous permettent de valider le test de cytotoxicité :

- Les absorbances doivent être supérieures à 0,2.
- La variation d'absorbance entre les deux colonnes de blanc ne doit pas dépasser 15%.

Pour les expériences *in vitro,* les hydrogels de l'exemple 1.7 (préparé avec le HA oxydé à température ambiante) sont fabriqués sous poste de sécurité microbiologique (PSM) en utilisant des tampons stériles et en utilisant des moules rincés avec un mélange éthanol:eau 70 :30 puis séchés à l'étuve à 60°C.

Exemple 4.2 : Stérilité des hydrogels de l'exemple 1.7 (préparé avec le HA oxydé à température ambiante)

Pour l'évaluation de la stérilité des hydrogels fabriqués sous PSM, les bouteilles contenant les hydrogels immergés dans du milieu de culture lors du test *in vitro* ont été laissées volontairement ouvertes (bouchons dévissés) afin de laisser passer l'air ambiant.

Le milieu de culture contient tous les composants nécessaires au développement de microorganismes (glucose, sérum, facteur de croissance ...). De plus les conditions de culture (37°C, 5% $CO_2$) sont favorables au développement bactérien et fongique. Une observation des hydrogels et du milieu d'extraction au microscope à la fin des tests *in vitro* permet de détecter une éventuelle contamination et donc de valider la stérilité de l'hydrogel.

Pour tous les hydrogels, aucune contamination bactérienne ou fongique n'a été observée au niveau du milieu de biodégradation, ce qui nous permet d'affirmer que les hydrogels sont fabriqués dans des conditions stériles et que le travail sous PSM permet à lui seul de garantir la stérilité.

Exemple 4.3 : biodégradabilité de l'hydrogel G1 et de l'hydrogel G2 de l'exemple 1.7 (préparés avec le HA oxydé à température ambiante)

Pour l'étude de la biodégradabilité de l'hydrogel G1 et de l'hydrogel G2 en milieu de culture cellulaire, des photos sont prises à différents temps du test *in vitro* afin d'étudier visuellement l'évolution de la structure et la détérioration des hydrogels.

L'étude de la biodégradation montre que la structure de l'hydrogel G1 se maintient mieux sur 29 jours que l'hydrogel G2 qui forme des cristaux à partir du 19ème jour. Au 29ème jour, l'hydrogel G2 s'est fragmenté alors que l'hydrogel G1, bien que plus étalé reste très maniable. A noter que G1 est plus teinté en jaune, ce qui démontre que le milieu de culture (jaune) pénètre plus dans l'hydrogel G1 que dans G2.

Evaluer la biodégradation des hydrogels pendant 29 jours en milieu de culture cellulaire nous informe sur la dégradation de l'hydrogel une fois implanté. L'hydrogel G2 se dégrade partiellement à partir du jour 19 en milieu de culture (pas complètement dégradé à jour 29), alors que l'hydrogel G1 reste stable au moins 29 jours en milieu de culture. La biodégradation très lente de G1 est plus adaptée à une implantation *in vivo* longue durée, alors que G2 est

plus intéressant pour une implantation plus courte.

Exemple 4.4 : cytotoxicité sur 29 jours des hydrogels de l'exemple 1.7 (préparés avec le HA oxydé à température ambiante)

Un test de cytotoxicité sur 29 jours a ensuite été réalisé sur les hydrogels G1 et G2. Le seuil de toxicité est à 70%.
L'hydrogel G1 ne présente aucune cytotoxicité sur 29 jours (tableau 6).

[Tableau 7]

Tableau 7 : Résultats du test de cytotoxicité sur 29 jours de l'hydrogel G1

| | Viabilité cellulaire (%) |
|---|---|
| contrôle (milieu de culture + cellules) | 100 |
| contrôle positif de toxicité (polyuréthane 0.1% de zinc) | 5 |
| contrôle négatif de toxicité (polyéthylène) | 101 |
| J1 avec le gel G1 | 93 |
| J5 avec le gel G1 | 102 |
| J8 avec le gel G1 | 122 |
| J12 avec le gel G1 | 116 |
| J15 avec le gel G1 | 118 |
| J19 avec le gel G1 | 126 |
| J22 avec le gel G1 | 119 |
| J26 avec le gel G1 | 123 |
| J29 avec le gel G1 | 119 |

L'hydrogel G2 ne présente aucune cytotoxicité sur 29 jours (Tableau 7). Les viabilités observées pour l'hydrogel G2 sont légèrement supérieures à celles obtenues pour G1.

[Tableau 8]

Tableau 8 : Résultats du test de cytotoxicité sur 29 jours de l'hydrogel G2

| | Viabilité cellulaire (%) |
|---|---|
| contrôle (milieu de culture + cellules) | 100 |
| contrôle positif de toxicité (polyuréthane 0.1% de zinc) | 2 |
| contrôle négatif de toxicité (polyéthylène) | 97 |
| J1 avec le gel G2 | 125 |
| J5 avec le gel G2 | 121 |
| J8 avec le gel G2 | 122 |
| J12 avec le gel G2 | 124 |
| J15 avec le gel G2 | 127 |
| J19 avec le gel G2 | 128 |
| J22 avec le gel G2 | 134 |

(suite)

| | Viabilité cellulaire (%) |
|---|---|
| J26 avec le gel G2 | 125 |
| J29 avec le gel G2 | 124 |

L'évaluation de la cytotoxicité sur 29 jours pour les hydrogels G1 et G2 montre que les deux gels ne sont pas cytotoxiques sur cette longue durée.

Example 5 (comparatif) : comparaison avec un gel de HA obtenu par oxydation du HA et réticulé par le Butyl Diglycidyl Ether (BDDE) (nommé « hydrogel HA-epoxy »):

Le HA (low HA, 100 mg/mL) est dissous dans une solution fraiche de NaOH 0,2 M sous agitation pendant 3h, jusqu'à l'obtention d'un pH de 12-13. 97 μL/mL de BDDE (liquide) est ajouté à la solution de HA et le mélange est agité pendant 10 min à température ambiante. Le mélange HA/BDDE est centrifugé pour éliminer les bulles d'air puis coulé dans des moules en téflon. Les gels sont mis à l'étuve à 40°C pendant 5 h pour accélérer le processus de réticulation, puis à température ambiante pendant la nuit.

Des hydrogels cohésifs sont obtenus, démoulés, et lavés/gonflés dans une solution de NaCl 0.9 w/v pendant au moins 1 semaine. Ils sont enfin stockés à 4°C.

Un test *in vitro* (stérilité, biodégradation, cytotoxicité) est réalisé avec le gel HA-epoxy obtenu, selon le même protocole que celui décrit précédemment.

Le gel est stérile et n'est pas dégradé après 29 jours dans le milieu de culture.

Le gel HA-epoxy présente de la toxicité (notamment à J22) après incubation dans le milieu de culture cellulaire (tableau 8). Le seuil de toxicité est à 70%. Contrairement aux gels G1 et G2, l'hydrogel HA-epoxy est cytotoxique.

[Tableau 9]

Tableau 9 : Résultats du test de cytotoxicité sur 29 jours de l'hydrogel G2

| | Viabilité cellulaire (%) du gel HA-epoxy (comparatif) | |
|---|---|---|
| | Essai 1 | Essai 2 |
| contrôle (milieu de culture + cellules) | 100 | 100 |
| contrôle positif de toxicité (polyuréthane 0.1% de zinc) | 2 | 1 |
| contrôle négatif de toxicité (polyéthylène) | 95 | 95 |
| J2 avec le gel HA-epoxy | 95 | 53 |
| J5 avec le gel HA-epoxy | 28 | 75 |
| J8 avec le gel HA-epoxy | 32 | 81 |
| J12 avec le gel HA-epoxy | 88 | 77 |
| J15 avec le gel HA-epoxy | 70 | 74 |
| J19 avec le gel HA-epoxy | 56 | 63 |
| J22 avec le gel HA-epoxy | 1 | 0 |
| J26 avec le gel HA-epoxy | 91 | 83 |
| J29 avec le gel HA-epoxy | 81 | 58 |
| BDDE 10% | 80 | 77 |
| BDDE 1% | 10 | 3 |

Exemple 6 : Tests *in vivo* des hydrogels

Exemple 6.1 : Biocompatibilité de l'hydrogel G1 après implantation chez le rat

La biocompatibilité, la biodégradation de l'hydrogel G1, ainsi que son impact sur l'inflammation post-chirurgicale, a été évaluée par implantation chez le rat pour une durée de 3 mois.

Le protocole expérimental était le suivant :
Toutes ces expériences ont été effectuées après acceptation du protocole d'expérimentation animale sous le numéro d'autorisation de projet 2017032709524056_v5#9502. Cette étude *in vivo* a été faite selon la norme ISO 10993-6:2016 qui spécifie les méthodes d'essai permettant d'évaluer les effets locaux après implantation de biomatériaux destinés à être utilisés dans des dispositifs médicaux.

Deux types de chirurgies ont été réalisées :

- Des craniotomies qui consistent en la découpe de la boîte crânienne et un soulèvement de la dure-mère pour une apposition des hydrogels en sub-cortical (feuillet). (6 rats implantés avec le gel G1)
- Des cortectomies qui consistent à découper la boîte crânienne, soulever la dure-mère puis faire une exérèse au niveau du cortex. L'implantation de l'hydrogel se fait directement dans la cavité d'exérèse (cylindre). (6 rats implantés avec le gel G1)

Pour cette étude *in vivo,* nous avons utilisé différents types de contrôles :

- Contrôle positif RM-B (Feuillet) : Polyurethane film containing 0.25% zinc dibuthyldithiocarbamate (ZDBC) - Selon la norme ISO 10993_6 (Hatano research) (3 rats)
- Contrôle positif RM-F (Cylindre) : Polyurethane rod containing 0.75% ZDEC - Selon la norme ISO 10993_6 (Hatano research) (3 rats)
- Contrôle négatif RM-C (Feuillet) : High density polyethylene film - Selon la norme ISO 10993_6 (Hatano research) (3 rats)
- Contrôle négatif RM-E (Cylindre) : High density polyethylene rod - Selon la norme ISO 10993_6 (Hatano research) (3 rats)
- Contrôle chirurgical : Craniotomie et/ou cortectomie effectuée sans implantation. Ce contrôle nous permet d'observer l'effet de la chirurgie sans apport de l'hydrogel. (3 rats)
- Contrôle cage (Sham) : Animaux non opérés du même lot et hébergés dans les mêmes conditions que les animaux de l'étude. (3 rats)

Craniotomie

- Induction de l'animale par anesthésie gazeuse.
- Incision médiane du scalp et résection des tissus sous cutanés
- Découpe et retrait du volet osseux (rectangle découpé dans la boîte crânienne)
- Retrait de la dure-mère
- Découpe au scalpel d'un feuillet de l'hydrogel de 3x5mm et de 1mm d'épaisseur
- Dépôt de l'hydrogel (feuillet) en position épi-corticale
- Scellement de la pièce osseuse
- Suture du scalp et désinfection.

La douleur post opératoire est prise en charge par l'administration en intra musculaire de 0,1mg.Kg de Buprénorphine (Vetergesic®).

Cortectomie

- Induction de l'animal par anesthésie gazeuse.
- Incision médiane du scalp et résection des tissus sous cutanés
- Découpe et retrait du volet osseux (rectangle découpé dans la boîte crânienne)
- Retrait de la dure-mère

- Réalisation d'une cortectomie (à l'aide d'un micro manipulateur supportant un "emporte-pièce" tissulaire)
- Découpe (à l'aide du micromanipulateur supportant l'emporte-pièce utilisé pour la cortectomie) d'un cylindre dans l'hydrogel de 1 mm de diamètre et 1 mm de hauteur
- Dépôt de l'hydrogel (cylindre) dans la cavité de cortectomie
- Scellement de la pièce osseuse
- Suture du scalp et désinfection.

La douleur post opératoire est prise en charge par l'administration en intra musculaire de 0,1mg.Kg de Buprénorphine (Vetergesic®).

Aucun problème majeur n'a été rencontré lors des chirurgies qui ont été effectuées par la même personne pour toutes les chirurgies.

Exemple 6.2 : Suivi des rats pendant les 3 mois d'implantation de l'hydrogel G1 : poids, comportement, apparence physique

Suite aux chirurgies, les rats sont suivis pendant les 3 mois d'hébergement, avec un suivi quotidien des animaux selon l'application de score et de point limite défini ci-dessous.

Les animaux seront examinés quotidiennement et un score clinique leur sera attribué en fonction de la grille ci-dessous. Tout score supérieur ou égal à 3 entrainera l'euthanasie de l'animal au détriment du protocole expérimental.

Changement de poids corporel initial :

0 Normal
1 Perte de poids < 10%
2 Perte de poids entre 10 et 15%
3 Perte de poids > 20%

Apparence physique :

0 Normale
1 Manque de toilettage
2 Poil ébouriffé, écoulement nasal/oculaire
3 Poil très ébouriffe, posture anormale

Comportement :

0 Normal
1 Changements mineurs : démarche boitante, protection de la plaie
2 Anormal : mobilité réduite, inactif
3 Vocalisations, auto-mutilation, très agité ou immobile

Ce suivi comprend des pesées de chaque rat, au minimum une fois par semaine. Les rats sont tous pesés selon le même protocole et sur la même balance tout au long des 3 mois d'hébergement. Un rat avec cortectomie + implantation de l'hydrogel G1 a perdu 16% de son poids pendant la semaine 8 après implantation, puis a retrouvé son poids de départ en 4,5 jours (complètement normal de la semaine 9 jusqu'à la fin de l'étude). Tous les autres rats ont eu des courbes de poids constantes. Le comportement des rats (activité, mouvements) ainsi que leur apparence physique (état du poil, posture) sont restés normaux tout au long de cette étude *in vivo,* ne traduisant aucun mal-être ou souffrance.

Exemple 6.3 : Suivi des rats pendant les 3 mois d'implantation de l'hydrogel G1 : IRM

Afin d'observer la dégradation de l'hydrogel G1 *in vivo,* nous avons fait passer des IRM à deux rats, l'un implanté avec un gel en feuillet, l'autre avec un gel en cylindre. Les IRM ont été faites 6 jours, 1 mois, 2 mois et 3 mois après implantation,. L'imagerie T2 est faite sur un IRM Bruker 4,7 Tesla en coupe coronale.

Les IRM passées sur le rat implanté avec l'hydrogel G1 déposé en feuillet après une craniotomie montrent que l'hydrogel G1 est toujours présent (hypersignal) après 3 mois d'implantation. Les dimensions de l'hydrogel G1 ne varient pas dans le temps, le temps de biodégradation de l'hydrogel est donc supérieur à 3 mois.

Les IRM passées sur le rat implanté avec l'hydrogel G1 déposé en cylindre après une craniotomie et une

cortectomie montrent que l'hydrogel est toujours présent (hypersignal) après 3 mois d'implantation. Les dimensions de l'hydrogel G1 ne varient pas dans le temps, bien qu'il occupe toute la cavité corticale à 3 mois (étalement du gel).

Le temps de biodégradation de l'hydrogel est donc supérieur à 3 mois.

Exemple 6.4 : Suivi des rats pendant les 3 mois d'implantation de l'hydrogel G1 : histologie

A la fin de l'étude (3 mois), les rats sont euthanasiés par exsanguination afin de préserver au mieux les cerveaux pour l'étude des tissus par histologie :

1) Anesthésie fixe Exagon (injection intra-péritonéale),
2) Perfusion intracardiaque de NaCl 0,9% (200 ml),
3) Perfusion intracardiaque de formol (400 ml),
4) Prélèvement de la pièce anatomique (cerveau).

Le protocole de préparation des cerveaux à l'histologie est le suivant :

- Fixation du cerveau dans du formol
- Imprégnation en sucrose 15%
- Imprégnation en sucrose 30 %
- Congélation de la pièce anatomique en vapeur d'azote liquide
- Coupe flottantes de 35 μm réalisées au cryostat et déposer dans une solution de cryoconservation à -20°C.

Coloration des coupes de cerveaux de rats au Crésyl Violet

La coloration au crésyl violet est une méthode de coloration histologique particulièrement utilisée pour révéler l'architecture cellulaire des tissus nerveux dont elle marque les corps cellulaires et tout particulièrement le réticulum endoplasmique rugueux. Le protocole utilisé est le suivant :

- Dégraissage : bains successifs de différentes concentrations d'éthanol
- Lavage à l'eau
- Coloration des coupes dans une solution de crésyl violet
- Fixation à l'éthanol 70% et 95%
- Lavage à l'eau
- Immersion des lames dans une solution de différenciation
- Lavage à l'eau
- Succession de bain d'éthanol de différentes concentrations
- Bain de xylène

Les acquisitions sont réalisées sur un microscope à fond clair.

Le contrôle Sham2 est le contrôle cage (non opéré). Les contrôles chirurgicaux sont des rats ayant subi les mêmes chirurgies craniotomie et cortectomie mais sans implantation de l'hydrogel. Ces contrôles nous permettent d'évaluer l'impact de la chirurgie sur la structure tissulaire et de la comparer avec les animaux implantés avec l'hydrogel et ainsi de déterminer l'action de l'hydrogel.

Les rats contrôle chirurgical craniotomie montrent que le retrait du volet osseux et de la dure-mère entraine un soulèvement de la partie supérieure du cortex. Nous observons aussi un épaississement de la limitante gliale lié au contact entre le volet osseux (solide) et le tissu cérébral (2KPa). L'acte de chirurgie craniotomie entraine donc des contraintes mécaniques, au niveau du tissu cérébral, liées à une différence de rigidité entre deux surfaces en contact.

Le rat contrôle chirurgical cortectomie montre les mêmes déformations de structure étant donné qu'une craniotomie est pratiquée avant la cortectomie. En plus du soulèvement de la partie supérieure du cortex, nous pouvons observer la cavité créée par le prélèvement d'un cylindre dans les premières couches corticales. La cortectomie entraine souvent un soulèvement des structures qui se situent sous le prélèvement comme l'hippocampe et le corpus calleux. Nous observons aussi un épaississement au niveau des bordures de la cavité corticale lié à la découpe des tissus lors de la chirurgie.

Le contrôle positif contient du zinc, toxique pour les tissus qui entraine une forte inflammation, alors que le contrôle négatif est non toxique et par conséquent n'entrainera pas d'inflammation liée à la toxicité du matériau implanté. Les résultats obtenus sur les rats implantés avec les contrôles négatifs (feuillet et cylindre) sont similaires aux résultats obtenus sur les contrôles chirurgicaux (déformation de la structure supérieure du cortex et épaississement de la limitante lors des craniotomies, lié aux contraintes mécaniques).

Les résultats obtenus sur les rats implantés avec les contrôles positifs (feuillet et cylindre) montrent une nécrose au niveau des tissus en contact avec le feuillet et le cylindre. Le dégagement de substances toxiques au niveau des tissus a entrainé une forte inflammation et donc un tissu nécrotique.

Les rats implantés avec le feuillet d'hydrogel G1 suite aux craniotomies présentent les mêmes changements de structure que les contrôles chirurgicaux. Pas de nécrose ou de zone d'inflammation prononcée.

Les rats implantés avec le cylindre d'hydrogel G1 suite aux craniotomies et cortectomies présentent les mêmes changements de structure que les contrôles chirurgicaux. Nous avons conservé l'hydrogel dans la cavité pour le rat N°9.

Lorsque nous regardons le site d'insertion de l'hydrogel et l'hydrogel conservé dans la cavité après prélèvement, nous pouvons voir que le gel est colonisé par des cellules après trois mois d'implantation.

Pour conclure sur cette étude, nous observons un changement dans la structure des tissus lorsqu'une craniotomie est pratiquée (soulèvement de couche externe supérieur du cortex), cette déformation est liée au retrait du volet osseux et de la dure-mère. Lors d'une cortectomie, une deuxième déformation est observée au niveau des structures qui se situent sous la cortectomie.

L'implantation de l'hydrogel G1 n'entraine pas de changement supplémentaire de la structure et les différentes couches du cortex sont conservées. Nous notons tout de même un apport de cellules au niveau de la limitante gliale, seulement pour les rats implantés avec l'hydrogel G1. Il est probable que l'acide hyaluronique, l'un des principaux constituant de la matrice extracellulaire impliqué dans la migration et la division cellulaire, ait joué un rôle dans l'augmentation du nombre de cellules au niveau de la zone implantée.

L'implantation de l'hydrogel G1 n'entraine pas de nécrose ou de forte inflammation, ces constatations seront vérifiées lors des études complémentaires d'immunohistochimie GFAP et Iba1.

Immunohistochimie SMI71/LAM ; GFAP ; Iba1

Un immuno-marquage SMI 71/Laminine a ensuite été effectué. La protéine endothéliale SMI 71 se situe principalement au niveau de la barrière hémato-encéphalique et/ou des jonctions neuro-vasculaires. La laminine est l'un des constituants majeurs de la matrice extracellulaire que l'on retrouve au niveau des endothéliums vasculaires. Ces deux marquages simultanés permettent de visualiser l'intégrité de la barrière hémato-encéphalique suite à l'implantation des hydrogels G1.

La protéine acide fibrillaire gliale (GFAP) est un filament intermédiaire présent dans certaines cellules gliales du système nerveux central, les astrocytes notamment.

Le marquage Iba1 est spécifique de la protéine Ionized calcium-binding adapter molecule 1 (Iba1). Parmi les cellules du cerveau, la protéine Iba1 est spécifiquement exprimée dans la microglie. L'activation microgliale est un autre type de réaction inflammatoire qui consiste en l'activation de la microglie, les cellules immunitaires du cerveau. Les cellules microgliales passe par différents stades d'activation, les prolongements des cellules s'élargissent de plus en plus jusqu'à former une sphère (stade macrophage).

Le protocole suivi pour les colorations SMI71/LAM, GFAP et Iba 1 est le suivant

JOUR 1

- Lavage des coupes dans du PBS 1X
- Perméabilisation membranaire PBS-Triton-NGS
- Saturation au PBS-BSA 3%
- Incubation avec l'anticorps primaire sur la nuit

JOUR 2

- Lavage PBS-BSA-Tween
- Incubation avec l'anticorps secondaire
- Lavage PBS-BSA-Tween
- Montage des coupes entre lame et lamelle

Les acquisitions sont réalisées au microscope confocal après une sélection des lames au microscope à épifluorescence.

Les anticorps utilisés ici sont :

- SMI 71 : spécifique de la protéine endothéliale SMI71, située au niveau de la barrière hémato-encéphalique et/ou des jonctions neuro-sanguines
- LAM : spécifique de la laminine, l'un des constituants majeurs de la matrice extracellulaire que l'on retrouve au

niveau des endothéliums vasculaires.

- GFAP : spécifique de la protéine acide fibrillaire gliale (GFAP) qui est un filament intermédiaire présent dans certaines cellules gliales du système nerveux central, les astrocytes notamment.
- Iba1 : spécifique de la protéine Ionized calcium-binding adapter molecule 1 (Iba1).

Parmi les cellules du cerveau, la protéine Iba1 est spécifiquement exprimée dans la microglie (cellules immunitaire du cerveau).

SMI71/LAM

Les résultats du contrôle cage (sham2) montrent la répartition de la protéine SMI 71 et de la laminine dans le cerveau d'un rat non opéré.

Le rat contrôle positif (Ctl+) cortectomie, implanté avec un barreau RM-E, présente un effondrement total du tissu et par conséquent de la barrière hémato-encéphalique alors que le contrôle positif feuillet maintient cette intégrité.

Les contrôles négatifs, feuillet et cylindre, sont similaires aux résultats obtenus sur le contrôle chirurgie et le contrôle cage (Sham 2).

L'expression de la protéine SMI 71 dans les coupes de cerveaux des rats n°2 (feuillet hydrogel G1) et n°9 (cylindre hydrogel G1) est comparable au contrôle chirurgie et au contrôle cage. L'expression de la laminine, une des protéines majoritaires de la matrice extracellulaire, est comparable à celle des contrôles pour les coupes du rat n°2 (feuillet hydrogel G1). Dans les coupes du rat n°9 (cylindre hydrogel G1), la laminine est exprimée au niveau des berges de la cavité corticale en comparaison du contrôle chirurgie qui ne présente pas de laminine au niveau de ses berges.

Les résultats obtenus pour les coupe de cerveaux des rats N°10 et 11 (cylindre hydrogel G1), montrent une surexpression de laminine dans les tissus environnant l'hydrogel. Cette surproduction peut être expliquée par la présence d'acide hyaluronique dans l'hydrogel. Des études bibliographiques ont montré que l'apport d'acide hyaluronique stimule la production de laminine par les cellules environnantes.

Pour conclure, l'implantation de l'hydrogel G1 que ce soit en condition feuillet (apposition sub-corticale) ou en condition cylindre (dépôt dans la cavité corticale) n'entraine pas de modification ou d'effondrement de la barrière hémato-encéphalique qui demeure intacte chez tous les rats testés.

L'augmentation de laminine dans l'environnement de l'hydrogel n'affecte pas la barrière hémato-encéphalique.

GFAP

Le marquage GFAP est spécifique de la protéine acide fibrillaire gliale (GFAP) qui est un filament intermédiaire présent dans certaines cellules gliales du système nerveux central, les astrocytes notamment.

La gliose astrocytaire est une réaction inflammatoire qui consiste en l'activation d'astrocytes au sein du tissu cérébral. Lorsque ces astrocytes sont activés, leur corps cellulaire s'arrondit et leurs prolongements cytoplasmiques s'épaississent.

Pour le contrôle cage (rat sham2), les astrocytes non activés sont répartis de manière homogène.

Le rat contrôle positif (Ctl+) cortectomie, implanté avec un barreau RM-E, présente un effondrement total du tissu menant à une nécrose (stade post-gliose astrocytaire) et par conséquent aucune gliose n'est observée.

Le contrôle positif feuillet présente une activation des astrocytes au niveau du contact avec le feuillet RM-B.

Les contrôles négatifs, feuillet et cylindre, sont similaires aux résultats obtenus sur le contrôle chirurgie et le contrôle cage (Sham 2).

Les coupes de cerveaux du rat n°2 (feuillet hydrogel G1) est comparable au contrôle chirurgie et ne présente pas de gliose astrocytaire. Dans les coupes du rat n°9 (cylindre hydrogel G1), une activation astrocytaire est observée autant sur le contrôle chirurgie que sur l'échantillon. Cette gliose est due à la chirurgie. L'hydrogel G1 seul contribue à réduire légèrement la gliose découlant de l'acte chirurgical.

Pour conclure, l'implantation de l'hydrogel G1, que ce soit en condition feuillet (apposition sub-corticale) ou en condition cylindre (dépôt dans la cavité corticale) n'entraine pas de gliose astrocytaire et donc de réaction inflammatoire. L'hydrogel G1 seul permet de réduire l'inflammation post-chirurgicale observée dans les conditions cylindre.

Iba1

Pour le contrôle cage (rat sham2), la microglie n'est pas activée.

Le rat contrôle positif (Ctl+) cortectomie, implanté avec un barreau RM-E, présente un effondrement total du tissu menant à une nécrose (stade post-activation microgliale) et par conséquent aucune activation de la microglie n'est observée. Le contrôle positif feuillet présente une activation microgliale au niveau du contact avec le feuillet RM-B.

Les contrôles négatifs, feuillet et cylindre, sont similaires aux résultats obtenus sur le contrôle chirurgie et le

contrôle cage (Sham 2).

Aucune activation microgliale n'est observée dans les coupes de cerveaux des rats n°2 (feuillet hydrogel G1) et n°9 (cylindre hydrogel G1), qui sont comparables aux contrôles chirurgie et au contrôle cage.

Pour conclure, l'implantation de l'hydrogel G1 que ce soit en condition feuillet (apposition sub-corticale) ou en condition cylindre (dépôt dans la cavité corticale) n'entraine pas d'activation microgliale et donc de réaction inflammatoire. L'implantation de l'hydrogel G1 n'entraine donc pas d'activation du système immunitaire.

Le tableau 9 ci-dessous récapitules les résultats d'histologie.

[Tableau 10]

Tableau 10 : Récapitulatif des résultats d'histologie

| | Coloration de Nissl | Gliose astocytaire marquage GFAP | Activation microgliale marquage Iba1 |
|---|---|---|---|
| Hydrogel feuillet HA | Changement de structure des couches corticales : non<br>Conservation des différentes couches corticales : oui<br>Signes de saignements ou de nécrose : non | Gliose astrocytaire : non | Activation microgliale : non |
| Contrôle chirurgie feuillet | Changement de structure des couches corticales : non<br>Conservation des différentes couches corticales : oui<br>Signes de saignements ou de nécrose : non | Gliose astrocytaire : non | Activation microgliale : non |
| Hydrogel cylindre HA | Changement de structure des couches corticales : non<br>Conservation des différentes couches corticales : oui<br>Signes de saignements ou de nécrose : non | Gliose astrocytaire : oui, réaction inflammatoire causé par la lésion chirurgicale. N'est pas liée à l'implantation de l'hydrogel. | Activation microgliale : non |
| Contrôle chirurgie cylindre | Changement de structure des couches corticales : non<br>Conservation des différentes couches corticales : oui<br>Signes de saignements ou de nécrose : non | Gliose astrocytaire : oui, réaction inflammatoire causé par la lésion chirurgicale. | Activation microgliale : non |

Conclusions sur l'étude de biocompatibilité de l'hydrogel G1 après implantation chez le rat

L'objectif de cette étude était de déterminer la biocompatibilité et la biostabilité de l'hydrogel G1 dans un modèle faisant intervenir des contingents cellulaires multiples (microglie, astrocytes, ...) communiquant et interagissant entre eux pour une réponse adaptée du tissu hôte. La complexité de ces interactions n'est pas reproductible *in vitro*. Ces études ont donc été menées chez le rat.

Les résultats démontrent que l'hydrogel G1 est parfaitement biocompatible *in vivo.* Il est peu dégradé et ne provoque aucune réaction inflammatoire pendant au minimum 3 mois d'implantation, et au contraire favorise la réduction de l'inflammation causée par l'acte chirurgical.

Exemple 7 : Hydrogel comprenant un agent anti-inflammatoire (kétorolac)

Exemple 7.1 inclusion de kétorolac dans le gel G1 pendant la réticulation du gel pour obtenir les gels G5 (G5-2.5mg, G5-5mg, G5-10mg)

Les gels G5 sont obtenus en introduisant le kétorolac pendant la réticulation du gel. Ils sont obtenus dans des conditions similaires à celles de la synthèse du gel G1, mais en présence de kétorolac, et sont synthétisés avec le protocole suivant :

1/ A 1.5 mL de solution aqueuse de HA (HyActive 100 mg/mL) est ajouté 0.8 mL d'une solution de $NaIO_4$ à 106 mg/mL et le milieu réactionnel est agité durant une nuit à température ambiante. Les résidus de $NaIO_4$ n'ayant pas réagi sont alors inactivés par l'éthylène glycol (37 µL d'une solution comprenant 20 µL d'éthylene glycol et 100 µL d'eau), rajouté au milieu réactionnel et incubé pendant 1h30 à température ambiante. Une solution de HA oxydé est alors obtenue (notée HAox-Tamb).
2/ Dans un moule en Téflon, 500 µL de la solution de HAox-Tamb est ajoutée et mélangée rapidement avec 40 µL de solution d'agent de réticulation bis-oxyamine L1 (obtenue en mélangeant 10 µL de L1 avec 90 µL d'eau) et 2.4 (gel G5-2.4mg), 4.2 (gel G5-4.2mg) ou 6.8 mg (gel G5-6.8mg) de kétorolac en solution dans l'eau.
3/ Après gélification en une vingtaine de secondes, les hydrogels G5 obtenus sont démoulés puis lavés à l'eau (7 mL) 4 fois 45 minutes.

Les gels préparés selon ce protocole présentent un aspect homogène translucide, similaire à celui de G1, sauf le gel G5-6.8mg légèrement opaque/laiteux.
La concentration en kétorolac dans les eaux de lavage est analysée par HPLC.

- HPLC (Waters analytique, Alliance)
- Colonne: Symmetry C18 / 3,5 µm / 4,6×75 mm
- Programme :
    - Débit: 1mL/min
    - Voie A: ACN / 0,1% TFA - voie B: Eau / 0,1% TFA
    - 5% A à 100% A en 20'
    - Détection à 320 nm et 270 nm

A partir de ces données, nous pouvons en déduire la quantité de kétorolac restante dans le gel. Les données sont regroupées dans le Tableau 10.

[Tableau 11]

Tableau 11: Quantité de kétorolac présente dans les gels G5

| Gel | Kétorolac |
|---|---|
| gel G5-2.4mg | 270 µg (soit 11.1% de la quantité initiale) |
| gel G5-4.2mg | 476 µg (soit 11.3% de la quantité initiale) |
| gel G5-6.8mg | 1777 µg (soit 26.2% de la quantité initiale) |

Exemple 7.2 : diffusion du kétorolac depuis les gels G5

Les gels G5 obtenus dans l'exemple 7.1 sont placés dans 2 mL de solution de NaCl 0,9% w/v. Cette solution de NaCl est renouvelée à des intervalles de temps fixés et chaque solution est analysée en HPLC pour la détermination de la concentration en kétorolac relargué dans la solution à l'instant étudié.

Description HPLC dans l'exemple 7.1.

A partir de ces données, la quantité de kétorolac relarguée du gel en fonction du temps est calculée (Tableau 11).

[Tableau 12]

Tableau 12 : Quantité de kétorolac (en μg) relargué des gels G5 en fonction du temps.

| Temps (h) | G5-2.4mg | G5-4.2mg | G5-6.8mg |
|---|---|---|---|
| 0 | 0 | 0 | 0 |
| 2 | 124,99 | 240,44 | 654,206 |
| 16 | 239,79 | 427,55 | 1447,496 |
| 48 | 263,56 | 466,78 | 1711,106 |
| 72 | 268,31 | 474,46 | 1764,296 |
| 144 | 269,19 | 476,04 | 1774,206 |
| 168 | 269,34 | 476,33 | 1775,956 |
| 240 | 269,38 | 476,42 | 1777,276 |
| 312 | 269,38 | 476,43 | 1777,426 |
| 360 | 269,38 | 476,43 | 1777,446 |

On observe un relargage rapide, dans les 48 premières heures d'incubation du gel. L'inflammation est plus aigüe juste après chirurgie, et les 48 heures post-chirurgie sont celles pour lesquelles la délivrance d'anti-inflammatoire est la plus pertinente.

Exemple 7.3 : Inclusion de kétorolac dans le gel G1 après la réticulation du gel pour obtenir les gels G6 (G6-10mg, G6-20mg, G6-30mg, G6-40mg)

Les gels G6 sont obtenus en introduisant le kétorolac après la réticulation du gel G1, qui est gonflé dans une solution aqueuse de kétorolac pour l'introduction du principe actif par diffusion. Ils sont obtenus dans des conditions similaires à celles de la synthèse du gel G1, et sont synthétisés avec le protocole suivant :

1/ A 1.5 mL de solution aqueuse de HA (HyActive 100 mg/mL) est ajouté 0.8 mL d'une solution de NaIO4 à 106 mg/mL et le milieu réactionnel est agité durant une nuit à température ambiante. Les résidus de NaIO4 n'ayant pas réagi sont alors inactivés par l'éthylène glycol (37 μL d'une solution comprenant 20 μL d'éthylene glycol et 100 μL d'eau), rajouté au milieu réactionnel et incubé pendant 1h30 à température ambiante. Une solution de HA oxydé est alors obtenue (notée HAox-Tamb).
2/ Dans un moule en Téflon, sont mélangés 40 μL de solution d'agent de réticulation bis-oxyamine L1 (obtenue en mélangeant 10 μL de L1 avec 90 μL d'eau) et 500 μL de la solution de HAox-Tamb sont alors ajoutés.
3/ Après gélification, l'hydrogel obtenu (hydrogel G1 non lavé) est démoulé puis lavé à l'eau.
4/ Après le lavage à l'eau du gel formé G1, ce dernier est incubé dans 7 mL d'une solution de kétorolac dans l'eau pendant 48 heures (10 mg/mL pour le gel G6-10mg, 20 mg/mL pour le gel G6-20mg, 30 mg/mL pour le gel G6-30mg, ou 40 mg/mL pour le gel G6-40mg mis dans un volume de 7 mL d'eau). A la fin de cette période d'incubation, la solution aqueuse de kétorolac entourant le gel est éliminée par pipetage.

Les gels préparés selon ce protocole présentent un aspect homogène translucide, similaire à celui de G1.

La quantité de kétorolac absorbée dans les gels est calculée comme la différence entre la quantité de drogue introduite initialement et la quantité de drogue retirée dans la solution aqueuse pipetée après incubation du gel et dosée par HPLC.

Description HPLC dans l'exemple 7.1.

A partir de ces données, nous pouvons en déduire la quantité de kétorolac dans les gels G6 (Tableau 12).

[Tableau 13]

Tableau 13. Quantité de kétorolac présente dans les gels G6.

| Gel | kétorolac |
|---|---|
| gel G6-10mg | 500 µg (soit 5 % de la quantité initiale) |
| gel G6-20mg | 1300 µg (soit 6.5 % de la quantité initiale) |
| gel G6-30mg | 1800 µg (soit 6% de la quantité initiale) |
| gel G6-40mg | 2600 µg (soit 6.5 % de la quantité initiale) |

Exemple 7.4 : Diffusion du kétorolac depuis les gels G6

Les gels G6 obtenus dans l'exemple 7.1 sont placés dans 2 mL de solution de NaCl 0,9% w/v. Cette solution de NaCl est renouvelée à des intervalles de temps fixés et chaque solution est analysée en HPLC pour la détermination de la concentration en kétorolac relargué dans la solution à l'instant étudié.

Description HPLC dans l'exemple 7.1.

A partir de ces données, la quantité de kétorolac relarguée du gel en fonction du temps est calculée (Tableau 13).

[Tableau 14]

Tableau 14 : Quantité de kétorolac (en µg) relargué des gels G6 en fonction du temps.

| Temps (h) | G6-10mg | G6-20mg | G6-30mg | G6-40mg |
|---|---|---|---|---|
| 0 | 0 | 0 | 0 | 0 |
| 3 | 249,09 | 824,67 | 959,57 | 1590,95 |
| 5 | 467,06 | 1234,32 | 1678,46 | 2331,38 |
| 24 | 477,35 | 1285,63 | 1750,91 | 2500,51 |
| 48 | 498,47 | 1299,07 | 1796,78 | 2593,45 |
| 72 | 499,93 | 1300 | 1800 | 2600 |

On observe un relargage rapide, essentiellement dans les 10 premières heures d'incubation du gel.

L'inflammation est plus aigüe juste après chirurgie, et les 48 heures post-chirurgie sont celles pour lesquelles la délivrance d'anti-inflammatoire est la plus pertinente.

Exemple 8 : Préparation de gel à partir d'un HAox purifié, lyophilisé.

- Préparation d'une solution de HA oxydé (notée HAox-Tamb) :

Un volume de 10 mL d'une solution aqueuse de HA 10% (HyActive 100 mg/mL eau) est ajouté à un volume de 5,31 mL d'une solution de $NaIO_4$ (106 mg / mL eau) et le milieu réactionnel est agité durant une nuit à température ambiante. Les résidus de $NaIO_4$ n'ayant pas réagi sont alors inactivés par l'ajout d'un volume de 166 µL d'une solution d'éthylène glycol (20 µL dans 100µL eau) et le milieu incubé pendant 1h à température ambiante. Une solution de HA oxydé est alors obtenue.

- Purification d'une solution de HA oxydé (notée HAox-Tamb) :

La totalité de cette solution (15,4 mL) est alors mise dans des boudins dialyse de seuil de coupure 12-14000

g/mol (SpectraPor, Toth Sochiel) puis dialysée contre l'eau durant 7 jours.

La solution purifiée de HA oxydée est ensuite mise en lyophilisation (appareil Labconco modèle Freezone 3.5) et aboutit à l'obtention d'un solide blanc dont l'analyse en infra-rouge (appareil Shimadzu modèle MIRacle 10) montre la présence d'un pic à 1730 $cm^{-1}$ caractéristique au carbonyl C=O de la fonction aldéhyde CHO.

- Réticulation d'une solution purifiée de HA oxydé :

Plusieurs concentrations en HA oxydé en solution dans l'eau ont été préparées : 1, 3, 4, 5 et 6% (w/v).

En exemple, pour la concentration à 1%, 2 mg de HA oxydé sont mis en solution dans 200 µL d'eau et pour la concentration de 6%, 12 mg de HA oxydé dans 200 µL d'eau. Un volume de 16 µL de solution d'agent de réticulation oxyamine à 0,7% v/v est alors ajouté à chaque échantillon et les résultats de réticulation montrent que, dans les conditions testées, les gels ne se forment qu'à partir de 5% w/v en concentration en HA oxydé dans l'eau.

## Revendications

1. Composition polymérique comprenant un polymère dérivé d'acide hyaluronique comprenant des unités réticulées pour former ensemble au moins l'un des groupements de formule (I), (II) ou (III) suivantes :

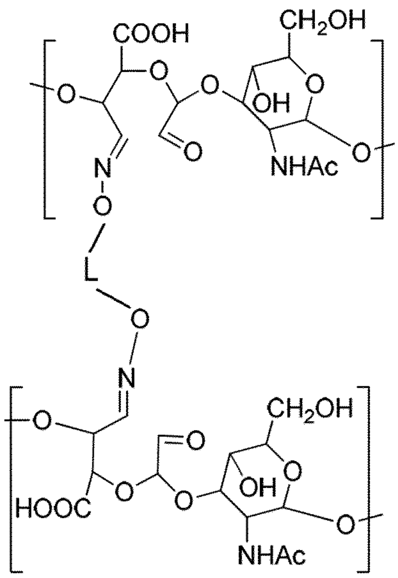
(I),

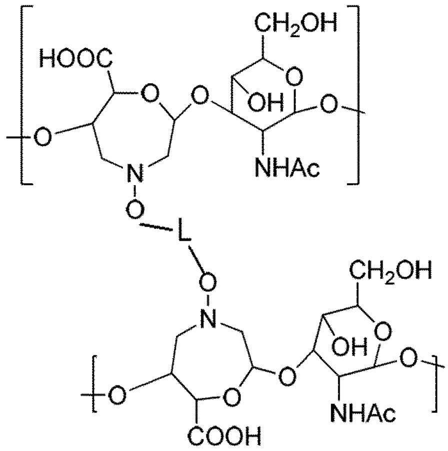
(II),

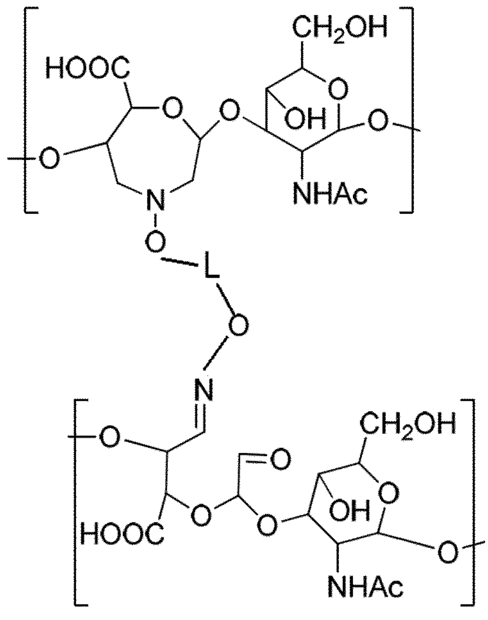

(III)

chaque groupement étant sous forme neutre ou de sel,
et dans lesquelles L est une chaîne hydrocarbonée comprenant de 2 à 10 atomes de carbone et éventuellement interrompue par un ou plusieurs groupes -O- ou -NH-.

2. Composition polymérique selon la revendication 1, dans laquelle le polymère dérivé d'acide hyaluronique polymère comprend des unités de formule (IV) :

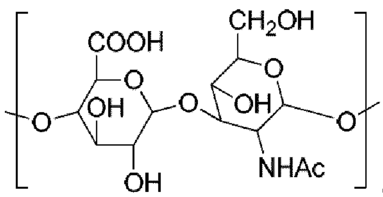

(IV)

chacune des unités de formule (IV) pouvant être sous forme neutre ou de sel.

3. Composition polymérique selon la revendication 1 ou 2, dans laquelle, dans les formules (I), (II) et (III), L représente un groupe de formule $-(CH_2)_2-O-(CH_2)_2-$, $-(CH_2)_2-O-(CH_2)_2-O-(CH_2)_2-$, $-(CH_2)_2-NH-(CH_2)_2-$, $-(CH_2)_2-NH-(CH_2)_2-NH-(CH_2)_2-$, $-(CH_2)_2-NH-(CH_2)_2-O-(CH_2)_2-$ ou $-(CH_2)_p-$ dans lequel p est un nombre entier de 2 à 10,

4. Composition polymérique selon la revendication 3, dans laquelle, dans les formules (I), (II) et (III), L représente le groupe $-(CH_2)_2-O-(CH_2)_2-$,

5. Composition polymérique selon l'une quelconque des revendications 1 à 4, comprenant un milieu aqueux, la composition polymérique se présentant sous forme d'hydrogel.

6. Composition polymérique sous forme d'hydrogel selon la revendication 5, dont le module d'élasticité G' est compris de 200 Pa à 20 000 Pa, plus préférentiellement de 500 Pa à 15 000 Pa, et encore préféré de 1000 à 12000 Pa tel que mesuré par rhéomètre sous cisaillement à 20°C.

7. Composition polymérique selon l'une quelconque des revendications 1 à 6, comprenant une protéine et/ou un agent thérapeutique, de préférence un agent anti-inflammatoire.

8. Procédé de préparation d'un polymère dérivé d'acide hyaluronique comprenant:

a) la fourniture d'un acide hyaluronique oxydé obtenu par oxydation d'acide hyaluronique par du périodate de sodium,
b) la réaction de l'acide hyaluronique oxydé avec un agent de réticulation de formule (XX) suivante :

$$H_2N\text{-}O\text{-}L\text{-}O\text{-}NH_2 \quad (XX)$$

dans laquelle L est une chaîne hydrocarbonée comprenant de 2 à 10 atomes de carbone et éventuellement interrompue par un ou plusieurs groupes -O- ou -NH-.

**9.** Kit comprenant :

- un premier compartiment comprenant un acide hyaluronique oxydé obtenu par oxydation d'acide hyaluronique par du périodate de sodium,
- un deuxième compartiment comprenant un agent de réticulation de formule (XX) telle que définie à la revendication 8.

**10.** Kit selon la revendication 9, se présentant sous forme d'une seringue d'injection (1) munie d'un piston (2) et d'une ouverture (3) sur laquelle une aiguille peut être fixée, l'ouverture (3) étant obturée par un premier opercule (4), ladite seringue d'injection (1) comprenant un premier compartiment (6) et un deuxième compartiment (7) séparés par un deuxième opercule (5) capable de se rompre lorsqu'une force est exercée sur le piston (2), où

- le premier compartiment (6) est localisé entre le premier opercule (4) et le deuxième opercule (5) et comprend un acide hyaluronique oxydé obtenu par oxydation d'acide hyaluronique par du périodate de sodium,
- le deuxième compartiment (7) est localisé entre le deuxième opercule (5) et le piston (2) et comprend un agent de réticulation de formule (XX) telle que définie à la revendication 8.

**11.** Kit selon la revendication 10, se présentant sous forme d'une seringue d'injection munie :

- d'une ouverture (13) sur laquelle une aiguille peut être fixée, l'ouverture (13) étant obturée par un premier opercule (14),
- d'un premier piston (12),
- d'un deuxième piston (11),
- d'un premier compartiment (16) comprenant un acide hyaluronique oxydé et lyophilisé obtenu par oxydation d'acide hyaluronique par du périodate de sodium puis lyophilisation,
- d'un deuxième compartiment (17) comprenant un agent de réticulation de formule (XX) telle que définie à la revendication 8,
- d'un troisième compartiment (18) comprenant un milieu aqueux,
- le premier compartiment (16) et le deuxième compartiment (17) étant séparés par un deuxième opercule (15) capable de se rompre lorsqu'une force est exercée sur le premier piston (12),
- le premier compartiment (16) et le troisième compartiment (18) étant séparés par un troisième opercule (19) capable de se rompre lorsqu'une force est exercée sur le deuxième piston (11),
- le deuxième compartiment (17) et le troisième compartiment (18) étant séparés par une paroi (20).

**12.** Composition polymérique sous forme d'hydrogel selon la revendication 5 ou 6, pour son utilisation :

- pour limiter la récidive d'une tumeur, de préférence du glioblastome, du cancer du sein, du pancréas, de la vessie, ou du sarcome chez un patient ayant subi une intervention chirurgicale d'exérèse de la tumeur, et/ou
- comme produit de comblement d'une cavité d'exérèse d'une tumeur, de préférence du glioblastome, du cancer du sein, du pancréas, de la vessie, ou du sarcome, et/ou
- pour le traitement d'une inflammation.

## Patentansprüche

**1.** Polymerzusammensetzung, umfassend ein von Hyaluronsäure abgeleitetes Polymer, umfassend Einheiten, die vernetzt sind, um zusammen mindestens eine der Gruppierungen von folgenden Formeln (I), (II) oder (III) zu bilden:

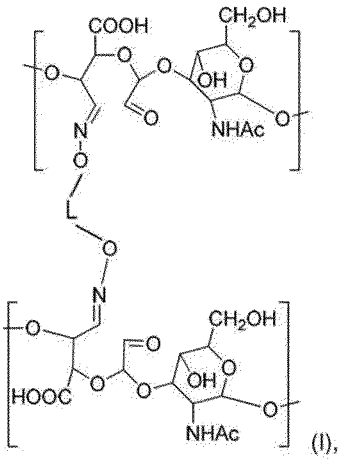
(I),

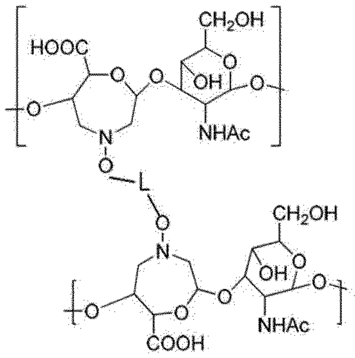
(II),

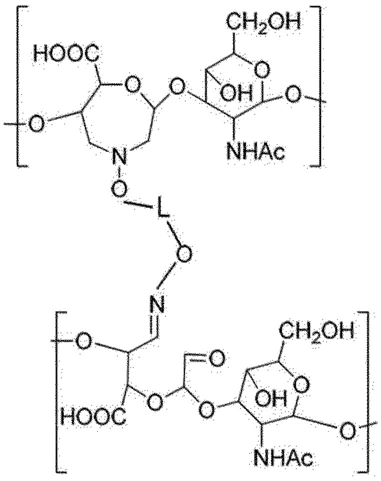
(III)

wobei jede Gruppierung in neutraler Form oder Salzform ist,
und wobei L eine Kohlenwasserstoffkette ist, umfassend 2 bis 10 Kohlenstoffatome und optional unterbrochen durch eine oder mehrere -O- oder -NH-Gruppen.

2. Polymerzusammensetzung nach Anspruch 1, wobei das von polymerer Hyaluronsäure abgeleitete Polymer Einheiten von Formel (IV) umfasst:

49

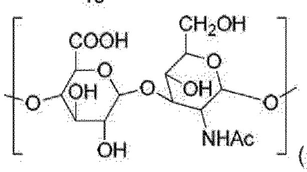
(IV)

wobei jede der Einheiten von Formel (IV) in neutraler Form oder Salzform sein kann.

3. Polymerzusammensetzung nach Anspruch 1 oder 2, wobei L in den Formeln (I), (II) und (III) eine Gruppe von Formel $-(CH_2)_2-O-(CH_2)_2-$, $-(CH_2)_2-O-(CH_2)_2-O-(CH_2)_2-$, $-(CH_2)_2-NH-(CH_2)_2-$, $-(CH_2)_2-NH-(CH_2)_2-NH-(CH_2)_2-$, $-(CH_2)_2-NH-(CH_2)_2-O-(CH_2)_2-$ oder $-(CH_2)_p-$darstellt, wobei p eine ganze Zahl von 2 bis 10 ist.

4. Polymerzusammensetzung nach Anspruch 3, wobei L in den Formeln (I), (II) und (III) die Gruppe $-(CH_2)_2-O-(CH_2)_2-$ darstellt.

5. Polymerzusammensetzung nach einem der Ansprüche 1 bis 4, umfassend ein wässriges Medium, wobei die Polymerzusammensetzung in Form von Hydrogel vorliegt.

**6.** Polymerzusammensetzung in Form von Hydrogel nach Anspruch 5, deren Elastizitätsmodul G' von 200 Pa bis 20.000 Pa, bevorzugter von 500 Pa bis 15.000 Pa und noch bevorzugter von 1000 bis 12.000 Pa ist, gemessen mit einem Rheometer unter Scherung bei 20 °C.

**7.** Polymerzusammensetzung nach einem der Ansprüche 1 bis 6, umfassend ein Protein und/oder ein therapeutisches Mittel, vorzugsweise ein entzündungshemmendes Mittel.

**8.** Verfahren zur Herstellung eines von Hyaluronsäure abgeleiteten Polymers, umfassend:

a) Bereitstellen einer oxidierten Hyaluronsäure, die durch Oxidation von Hyaluronsäure mit Natriumperiodat erlangt wird,
b) Reagieren der oxidierten Hyaluronsäure mit einem Vernetzungsmittel von folgender Formel (XX):

$$H_2N\text{-}O\text{-}L\text{-}O\text{-}NH_2 \quad (XX)$$

wobei L eine Kohlenwasserstoffkette ist, umfassend 2 bis 10 Kohlenstoffatome und optional unterbrochen durch eine oder mehrere -O- oder -NH-Gruppen.

**9.** Kit, umfassend:

- eine erste Kammer, umfassend eine oxidierte Hyaluronsäure, die durch Oxidation von Hyaluronsäure mit Natriumperiodat erlangt wird,
- eine zweite Kammer, umfassend ein Vernetzungsmittel von Formel (XX), wie es in Anspruch 8 definiert ist.

**10.** Kit nach Anspruch 9, das in Form einer Injektionsspritze (1) vorliegt, die mit einem Kolben (2) und einer Öffnung (3), an der eine Nadel befestigt werden kann, versehen ist, wobei die Öffnung (3) durch einen ersten Deckel (4) verschlossen ist, die Injektionsspritze (1) umfassend eine erste Kammer (6) und eine zweite Kammer (7), die durch einen zweiten Deckel (5) getrennt sind, der brechen kann, wenn eine Kraft auf den Kolben (2) ausgeübt wird, wobei

- die erste Kammer (6) zwischen dem ersten Deckel (4) und dem zweiten Deckel (5) angeordnet ist und eine oxidierte Hyaluronsäure umfasst, die durch Oxidation von Hyaluronsäure mit Natriumperiodat erlangt wird,
- sich die zweite Kammer (7) zwischen dem zweiten Deckel (5) und dem Kolben (2) befindet und ein Vernetzungsmittel von Formel (XX), wie es in Anspruch 8 definiert ist, umfasst.

**11.** Kit nach Anspruch 10, das in Form einer Injektionsspritze vorliegt, die versehen ist mit:

- einer Öffnung (13), an der eine Nadel befestigt werden kann, wobei die Öffnung (13) durch einen ersten Deckel (14) verschlossen ist,
- einem ersten Kolben (12),
- einem zweiten Kolben (11),
- einer ersten Kammer (16), umfassend eine oxidierte und lyophilisierte Hyaluronsäure, die durch Oxidation von Hyaluronsäure mit Natriumperiodat und anschließende Lyophilisierung erlangt wird,
- einer zweiten Kammer (17), umfassend ein Vernetzungsmittel von Formel (XX), wie es in Anspruch 8 definiert ist,
- einer dritten Kammer (18), umfassend ein wässriges Medium,
- wobei die erste Kammer (16) und die zweite Kammer (17) durch einen zweiten Deckel (15) getrennt sind, der in der Lage ist, zu brechen, wenn eine Kraft auf den ersten Kolben (12) ausgeübt wird,
- wobei die erste Kammer (16) und die dritte Kammer (18) durch einen dritten Deckel (19) getrennt sind, der in der Lage ist, zu brechen, wenn eine Kraft auf den zweiten Kolben (11) ausgeübt wird,
- wobei die zweite Kammer (17) und die dritte Kammer (18) durch eine Wand (20) getrennt sind.

**12.** Polymere Zusammensetzung in Form eines Hydrogels nach Anspruch 5 oder 6 zur Verwendung:

- um das Wiederauftreten eines Tumors, vorzugsweise Glioblastom, Brust-, Bauchspeicheldrüsen-, Blasen- oder Sarkomkrebs, bei einem Patienten, der sich einer Tumorentfernungsoperation unterzogen hat, zu vermeiden, und/oder

- als Füllstoff für eine Exzisionshöhle eines Tumors, vorzugsweise Glioblastom, Brust-, Bauchspeicheldrüsen-, Blasen- oder Sarkomkrebs, und/oder
- für die Behandlung einer Entzündung.

## Claims

1. A polymeric composition comprising a polymer derived from hyaluronic acid having crosslinked units together forming at least one of the following groups of formula (I), II) or (III):

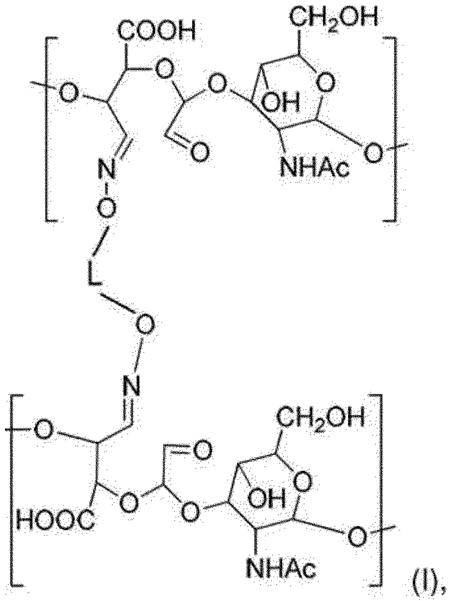
(I),

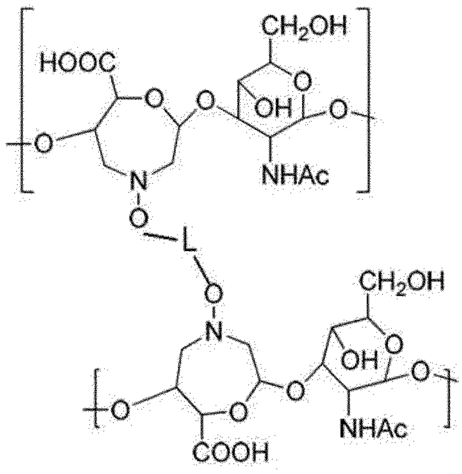
(II),

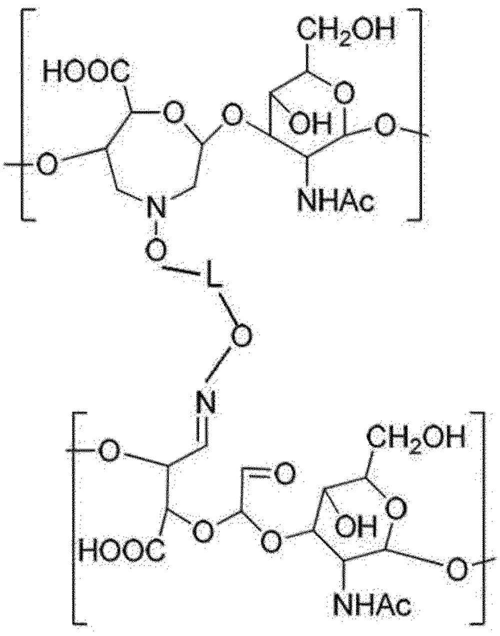
(III)

each group being in neutral or salt form,
and wherein L is a hydrocarbon chain having 2 to 10 carbon atoms and optionally interrupted by one or more groups -O- or - NH-.

2. The polymeric composition according to claim 1, wherein the polymer derived from hyaluronic acid comprises units

of formula (IV):

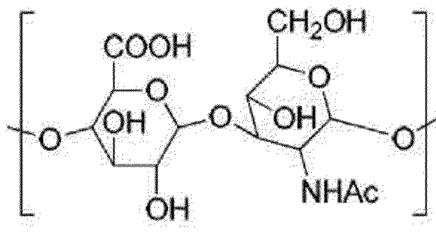

(IV)

each of the units of formula (IV) possibly being in neutral or salt form.

**3.** The polymeric composition according to claim 1 or 2 wherein, in formulas (I), (II) and (III), L is a group of formula $-(CH_2)_2-O-(CH_2)_2-$, $-(CH_2)_2-O-(CH_2)_2-O-(CH_2)_2-$, $-(CH_2)_2-NH-(CH_2)_2-$, $-(CH_2)_2-NH-(CH_2)_2-NH-(CH_2)_2-$, $-(CH_2)_2-NH-(CH_2)_2-O-(CH_2)_2-$ or $-(CH_2)_p-$where p is an integer of 2 to 10.

**4.** The polymeric composition according to claim 3 wherein, in formulas (I), (II) and (III), L is the group $-(CH_2)_2-O-(CH_2)_2-$.

**5.** The polymeric composition according to any of claims 1 to 4 comprising an aqueous medium, the polymeric composition being in hydrogel form.

**6.** The polymeric composition in hydrogel form according to claim 5 having an elastic modulus G' of 200 Pa to 20 000 Pa, more preferably from 500 Pa to 15 000 Pa, further preferably from 1 000 to 12 000 Pa, as measured by rheometer under shear at 20 °C.

**7.** The polymeric composition according to any of claims 1 to 6, comprising a protein and/or therapeutic agent, preferably an anti-inflammatory agent.

**8.** A method for preparing a polymer derived from hyaluronic acid, comprising:

a) providing an oxidized hyaluronic acid obtained by oxidation of hyaluronic acid by sodium periodate,
b) reacting the oxidized hyaluronic acid with a crosslinking agent of following formula (XX):

$H_2N-O-L-O-NH_2$ (XX)

where L is a hydrocarbon chain having 2 to 10 carbon atoms and optionally interrupted by one or more groups -O- or -NH-.

**9.** A kit comprising:

- a first compartment comprising oxidized hyaluronic acid obtained by oxidation of hyaluronic acid by sodium periodate,
- a second compartment comprising a crosslinking agent of formula (XX) as defined in claim 8.

**10.** The kit according to claim 9 in the form of an injection syringe (1) provided with a plunger (2) and an orifice (3) onto which a needle can be secured, the orifice (3) being closed by a first seal (4), said injection syringe (1) comprising a first compartment (6) and a second compartment (7) separated by a second seal (5) capable of breaking when a force is applied to the plunger (2), wherein;

- the first compartment (6) is located between the first seal (4) and second seal (5) and comprises oxidized hyaluronic acid obtained by oxidation of hyaluronic acid by sodium periodate,
- the second compartment (7) is located between the second seal (5) and the plunger (2) and comprises a crosslinking agent of formula (XX) as defined in claim 8.

**11.** The kit according to claim 10, in the form of an injection syringe provided with:

- an orifice (13) on which a needle can be secured, the orifice (13) being closed by a first seal (14),

- a first plunger (12),
- a second plunger (11),
- a first compartment (16) comprising oxidized and lyophilized hyaluronic acid obtained by oxidation of hyaluronic acid by sodium periodate, followed by lyophilization,
- a second compartment (17) comprising a crosslinking agent of formula (XX) as defined in claim 8,
- a third compartment (18) comprising an aqueous medium,
- the first compartment (16) and second compartment (17) being separated by a second seal (15) capable of breaking when a force is applied to the first plunger (12),
- the first compartment (16) and third compartment (18) being separated by a third seal (19) capable of breaking when a force is applied to the second plunger (11),
- the second compartment (17) and third compartment (18) being separated by a wall (20).

**12.** The polymeric composition in hydrogel form according to claim 5 or 6, for use thereof:

- to limit recurrence of a tumour, preferably in glioblastoma, in cancer of the breast, pancreas, bladder, or in sarcoma, in a patient having undergone surgery for excision of the tumour, and/or
- as product to fill a tumour excision cavity, preferably in glioblastoma, in cancer of the breast, pancreas, bladder, or in sarcoma, and/or
- to treat an inflammation.

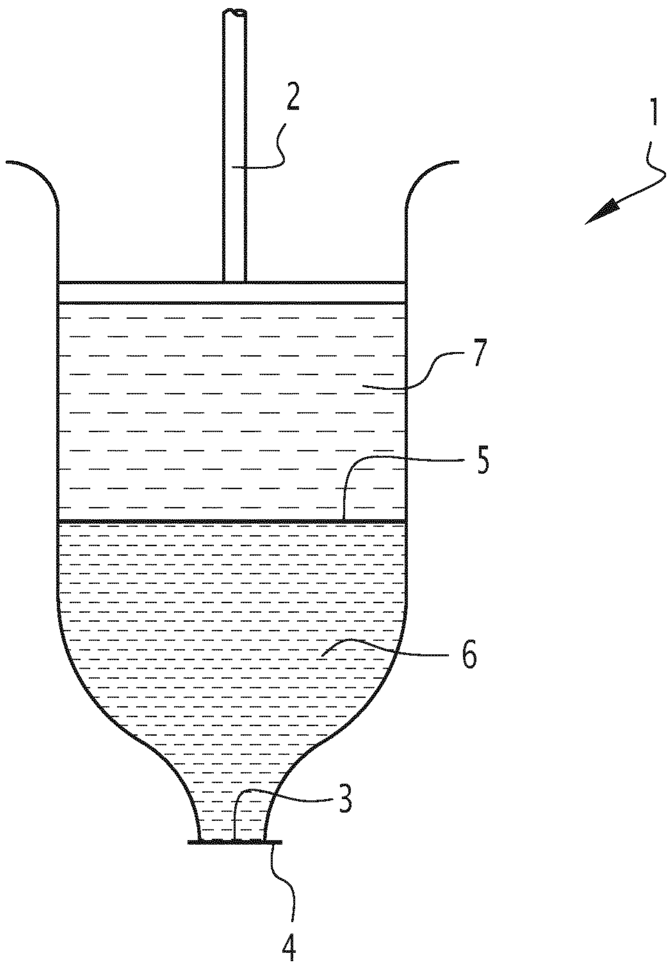


FIG.1

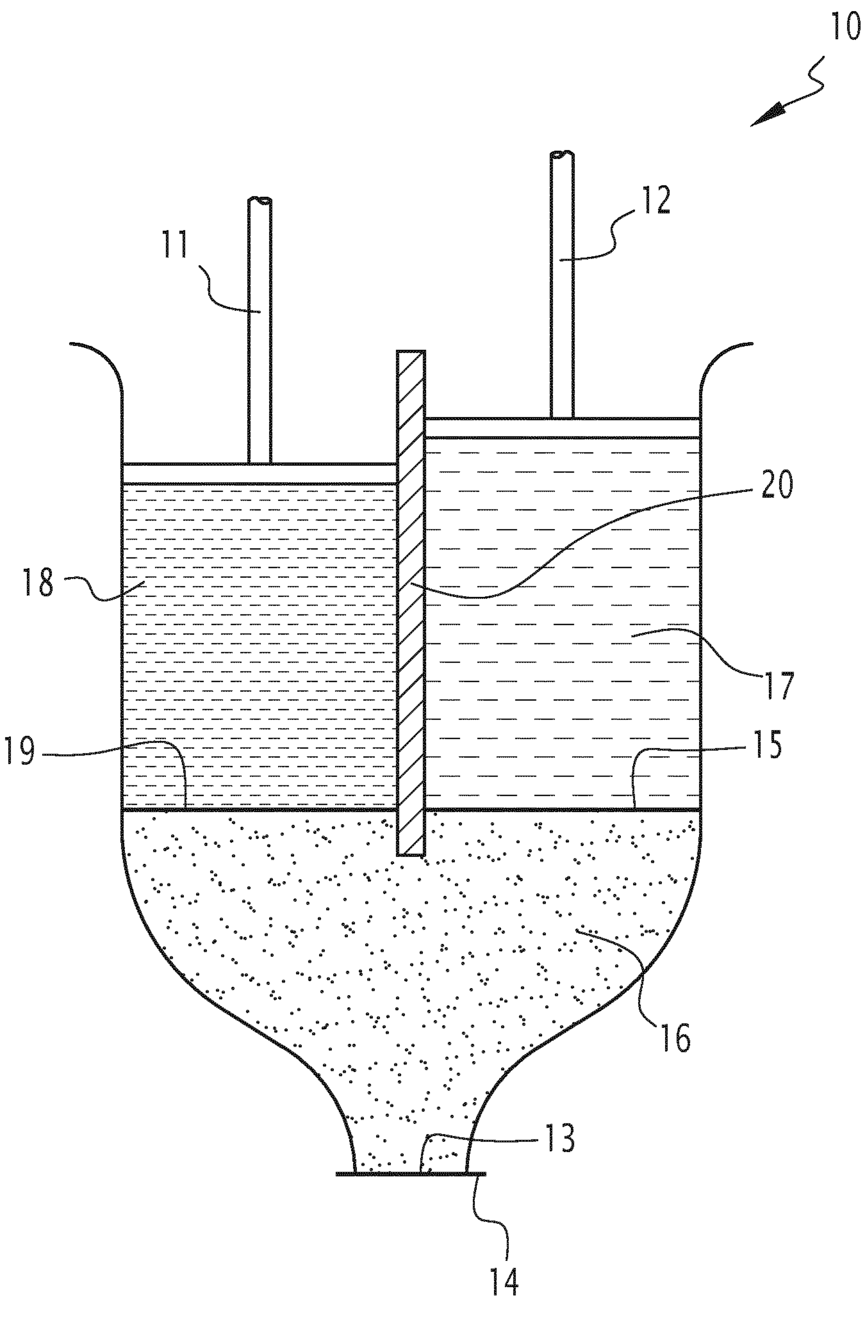


FIG.2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description

- **BAKER et al.** *Biomacromolecules,* 2017, vol. 18, 4373-4384